# EUROPEAN PATENT APPLICATION

(11) **EP 4 527 382 A1**
(43) Date of publication of application: **26.03.2025**
(21) Application number: 22942367.8
(22) Date of filing: 05.09.2022
(51) Int. Cl.: A61K 31/325, A61K 31/198, A61K 31/381, A61K 31/426, A61K 45/06, A61K 9/00, A61K 9/06, A61K 9/14, A61K 9/19, A61K 47/02, A61K 47/18, A61K 47/20, A61K 47/26, A61K 47/38, A61P 35/00, A61P 39/02, A23L 33/10

(54) **HIGH-STABILITY HEAVY METAL REMOVING COMPOSITION, USE AND DOSAGE FORM THEREOF, AND METHOD FOR PREPARING SAME**

(30) Priority: 17.05.2022 CN 202210531280
(71) Applicant: Guangdong Jianersheng Pharmaceutical Technology Co., Ltd., Zhuhai, Guangdong 519031 (CN)
(72) Inventor: TANG, Xiaojiang, Guangzhou, Guangdong 510663 (CN); HU, Wei, Guangzhou, Guangdong 510663 (CN); ZHONG, Zhiyong, Guangzhou, Guangdong 510663 (CN); ZHAO, Qile, Guangzhou, Guangdong 510663 (CN); PAN, Shutao, Guangzhou, Guangdong 510663 (CN); WANG, Xuemei, Guangzhou, Guangdong 510663 (CN); HUANG, Xiao, Guangzhou, Guangdong 510663 (CN); MAI, Dongmei, Guangzhou, Guangdong 510663 (CN); REN, Xuefeng, Guangzhou, Guangdong 510663 (CN); DONG, Guanghui, Guangzhou, Guangdong 510663 (CN)
(74) Representative: Meyer, Thorsten
(86) International application number: PCT/CN2022/116954
(87) International publication number: WO 2023/221338

(57) **Abstract**

Disclosed are a highly stable heavy metal-removing composition, use and a dosage form thereof, and a preparation method therefor, which belong to the field of medicine. The composition comprises: (A) 70-95wt% of a compound represented by formula (1) or a pharmaceutically acceptable salt thereof; (B) 1-25wt% of a compound represented by formula (2) or a pharmaceutically acceptable salt thereof; (C) 0.001-5wt% of an alkaline compound, which is at least one selected from the group consisting of a hydroxide of an alkali metal, a carbonate of an alkali metal, a bicarbonate of an alkali metal, a biphosphate of an alkali metal, a carboxylate of an alkali metal, and ammonium hydroxide.

## Description

### TECHNICAL FIELD

The present disclosure belongs to the field of medicine and relates to a highly stable heavy metal-removing composition, use of the composition in the preparation of a medicament for removing a heavy metal and free radicals, use of the composition in the preparation of an antineoplastic medicament, use of the composition in the preparation of a medicament for preventing and treating a related disease caused by a heavy metal, a medicament with the composition as the main component and corresponding dosage forms, and a preparation method of the composition.

### BACKGROUND ART

Dithiocarbamate (DTC) is a new class of complexing agent studied in the 80s of the last century, and the general structural formula is:.

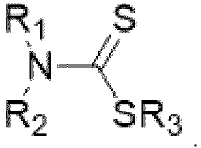

As a class of widely existing organic molecule, it has a variety of functional groups and heteroatoms, which makes it have strong metal bonding ability, so that it can effectively chelate heavy metals and remove NO free radicals in the body. Different DTC derivatives exhibit many biological and pharmaceutical properties, such as excellent anti-inflammatory, antibacterial, antioxidant and other activities, and have recently been reported as effective anticancer drugs and inhibitors of cell apoptosis.

GDTC (R₂ in DTC replaced by glucosyl) as a class of drugs that can remove heavy metals in kidney cells, has been the focus of research in this field. In 1989, Gale *et al.* replaced R1 with a methoxybenzyl group to obtain sodium 4-methoxybenzyl-D-glucosamine-N-dithiocarboxylate (4-Me), which has a good cadmium-removing effect on rats with chronic cadmium poisoning. In 1995, Professor Jinyuan Zhao *et al.,* replaced R1 with 6 different amino acids to obtain six new complexes, and it was found by cadmium-removing experiments in rats that the six new complexes have a certain cadmium-removing effect, all of which are better than the cadmium-removing effect of 4-Me, but because of their toxic side effects (in severe cases, they may cause death), significant irritation after injection and other reasons, so far they have not be applied in clinical practice. In 2005, Xiaojiang Tang *et al.* replaced R1 with methionine, and replaced R2 with glucosyl to obtain a new complex N-(2,3,4,5,6-pentahydroxyhexyl)-(N-disubstituted sodium formate)-L-sodium methionate (GMDTC), which has a significant removing effect on cadmium in the kidney and few toxic side effects; Xiaojiang Tang *et al.* proposed its action mechanism in 2016, that is, GMDTC uses SGLT2-GLUT2, the glucose transporter, to freely enter and exit renal tubular epithelial cells, thus achieving the purpose of removing heavy metals from kidney cells and laying a foundation for the application of such drugs. [Xiaojiang Tang et al., Mobilization and removing of cadmium from kidney by GMDTC utilizing renal glucose reabsorption pathway, Toxicology and Application Pharmacology 305 (2016) 143 - 152].

However, the stability of GDTC drugs has not been resolved, making GDTC drugs difficult to be prepared into a medicament; there are also problems in the compatibility stability, and GDTC drugs will hydrolyze in a solvent. Therefore, in animal experiments, a higher dose is often required to have a good effect on the removal of heavy metals. Meanwhile, due to poor stability, the production, transportation and storage costs of such drugs are high, the formulation preparation is difficult, and the validity period is short, resulting in limited practical applications.

The Chinese invention application CN108546242A discloses a dithiocarbamate compound and its use in the preparation of a heavy metal-removing medicament. The stability is improved by changing the molecular structure. However, its maximum stability period is only 14 hours, which still cannot meet the transportation and storage requirements in clinical applications.

The Chinese invention application CN108276319A discloses a new thiocompound and its use in the preparation of a heavy metal-removing medicament. Similarly, the stability is improved by changing the molecular structure. However, its maximum stability period is only 24 hours, which still cannot meet the transportation and storage requirements in clinical applications.

There is an urgent need to solve the stability problem of GDTC drugs so that they can meet the transportation and storage requirements in clinical applications, so as to achieve a large-scale clinical application as soon as possible and relieve the pain of patients.

### SUMMARY

After a long-term research on GDTC drugs, the inventors of the present disclosure broke away the traditional idea of improving stability by changing the molecular structure, and developed a composition in a different way, which greatly improves the stability under the premise of maintaining the heavy metal-removing effect of GDTC drugs, and can achieve long-term storage under conventional conditions. Meanwhile, due to the improved stability, the drug dose can be significantly reduced on the basis of achieving the same therapeutic efficacy, thus improving the safety of drug use and reducing the treatment cost.

The purpose of the present disclosure is to provide a highly stable heavy metal-removing composition, solve the stability problem of GDTC drugs, so that GDTC drugs can be prepared into various preparations, and can be widely used. The present disclosure also provides use of the composition in the preparation of a medicament for removing a heavy metal and free radicals, use of the composition in the preparation of an antineoplastic medicament, use of the composition in the preparation of a medicament for preventing and treating a related disease caused by a heavy metal, a medicament with the composition as the main component and corresponding dosage forms, and a preparation method of the composition. In order to achieve the above objectives, the specific technical solutions specifically adopted in the present disclosure are as follows.

The present disclosure provides a highly stable heavy metal-removing composition, comprising:
(A) 70-95wt% of a compound represented by formula (1) or a pharmaceutically acceptable salt thereof;
(B) 1-25wt% of a compound represented by formula (2) or a pharmaceutically acceptable salt thereof;
(C) 0.001-5wt% of an alkaline compound, which is at least one selected from the group consisting of a hydroxide of an alkali metal, a carbonate of an alkali metal, a bicarbonate of an alkali metal, a biphosphate of an alkali metal, a carboxylate of an alkali metal, and ammonium hydroxide;
where a combination of the compound represented by formula (1) and the compound represented by formula (2) is one selected from the combinations listed in the table below:

**Table 1: Molecular structures of compounds in formula (1) and formula (2).**

| SN | Formula (1) | Formula (2) |
|---|---|---|
| 1 | 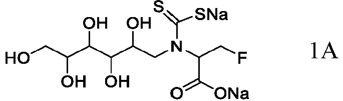 | 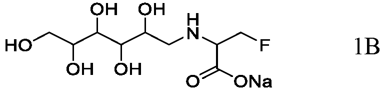 |
| 2 | 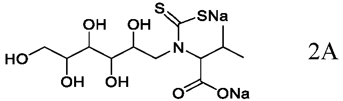 | 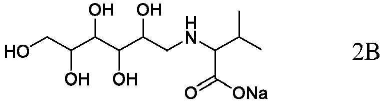 |
| 3 | 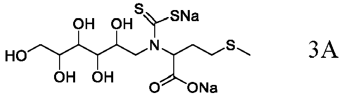 | 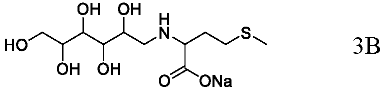 |
| 4 | 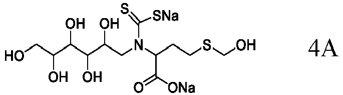 | 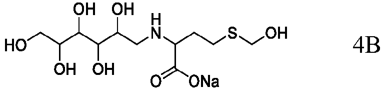 |
| 5 | 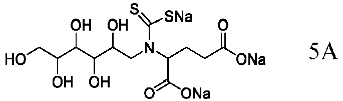 | 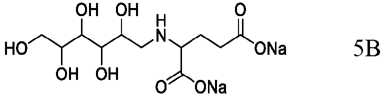 |
| 6 | 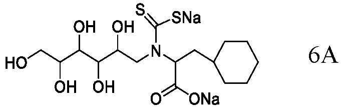 | 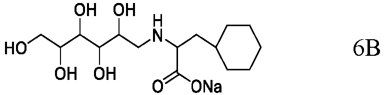 |
| 7 | 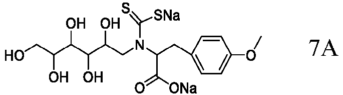 | 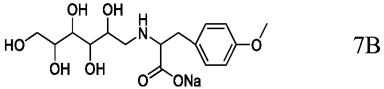 |
| 8 | 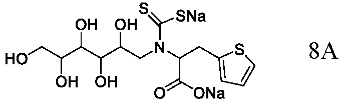 | 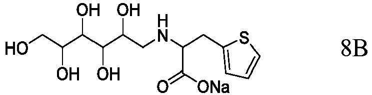 |
| 9 | 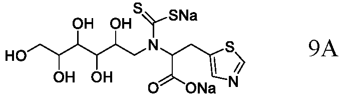 | 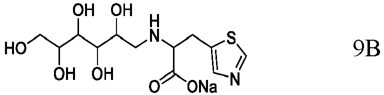 |
| 10 | 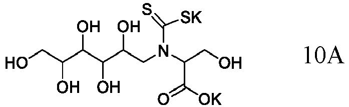 | 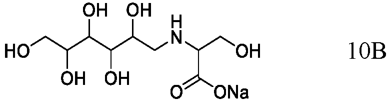 |
| 11 | 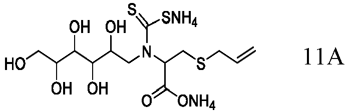 | 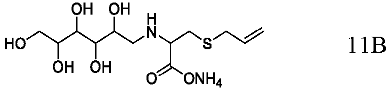 |

Preferably, the compound represented by formula (1) in the composition is: 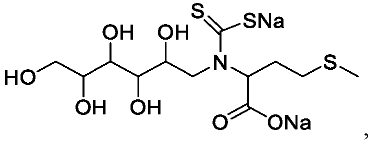 and
the compound represented by formula (2) is:

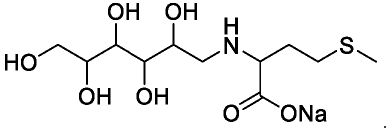

Preferably, a content of component (A) in the composition is 80-90wt%, and a content of component (B) in the composition is 1-12wt%.

Preferably, a ratio of a mass fraction of component (A) in the composition to a mass fraction of component (B) in the composition is in the range from 90:1 to 9:1. More preferably, the ratio of the mass fraction of component (A) in the composition to the mass fraction of component (B) in the composition is in the range from 80:1 to 13:1.

Preferably, the alkaline compound is at least one selected from the group consisting of sodium hydroxide, potassium hydroxide, sodium acetate, ammonium hydroxide, sodium carbonate, sodium bicarbonate, ammonium bicarbonate, potassium carbonate, potassium bicarbonate, dipotassium hydrogenphosphate, and disodium hydrogenphosphate.

Preferably, a pH value of a solution formed by dissolving 1mg of the composition in 1mL of water is 9.0~11.0.

The present disclosure provides use of the aforementioned composition in the preparation of a medicament or a functional food for removing a heavy metal or free radicals from a body, or in the preparation of a medicament for preventing or treating a related disease caused by heavy metal-excess and heavy metal-poisoning; the heavy metal is at least one selected from the group consisting of chromium, cobalt, arsenic, tin, cadmium, mercury, manganese, nickel, copper, thallium, technetium, uranium, bismuth, lead, iodine, palladium and platinum.

The present disclosure provides an antineoplastic medicament, comprising by weight: 1 portion of a heavy metal-containing antineoplastic drug, and 5-200 portions of the aforementioned composition. Preferably, the heavy metal-containing antineoplastic drug is selected from: a platinum-containing antineoplastic drug, a arsenic-containing antineoplastic drug, a ruthenium-containing antineoplastic drug or a tin-containing antineoplastic drug. More preferably, the heavy metal-containing antineoplastic drug is selected from: cisplatin, oxaliplatin, arsenic trioxide or iodine 125.

The present disclosure provides use of the aforementioned composition in the preparation of a medicament for reducing a toxic side effect of a heavy metal-containing drug; the heavy metal-containing drug comprises at least one selected from the group consisting of cisplatin, carboplatin, oxaliplatin, nedaplatin, bismuth potassium citrate, colloidal pectin bismuth, technetium 99 dimethyl bisphosphonate, technetium 99mTc, arsenic trioxide, iodine 125 and palladium 103. Preferably, the heavy metal-containing drug comprises at least one selected from the group consisting of cisplatin, bismuth potassium citrate and technetium 99mTC.

The present disclosure provides a powder injection, based on a total weight of the powder injection, comprising: 60-90wt% of the aforementioned composition; 9-40wt% of an excipient; and 0.001-10wt% of a complexing agent. Preferably, the excipient is at least one selected from the group consisting of mannitol, ammonium hydroxide, trehalose, sodium bicarbonate, sodium carbonate, potassium carbonate, sodium acetate, ammonium acetate and dipotassium hydrogenphosphate; more preferably, the excipient is at least one selected from the group consisting of mannitol and ammonium hydroxide; more preferably, the excipient is ammonium hydroxide, and ammonium hydroxide is present in the form of a complex or ammine in the powder injection. Preferably, the complexing agent is at least one selected from the group consisting of ethylenediaminetetraacetate (EDTA), dimercaptopropanol (BAL), and sodium dimercaptosuccinate (DMSA). More preferably, the complexing agent is EDTA.

The present disclosure provides an oral preparation, based on a total weight of the oral preparation, comprising: 35-65wt% of the aforementioned composition; 20-40wt% of a disintegrant; 0-5wt% of a flow aid; 0.1-5wt% of a lubricant; 0~1wt% of a film-coating premix; and 0.001-10wt% of a complexing agent. Preferably, the disintegrant is at least one selected from the group consisting of microcrystalline cellulose, sodium carboxymethylcellulose, sodium carboxymethyl starch, starch, and polyvinylpyrrolidone; the flow aid is at least one selected from the group consisting of talc, and colloidal silica; the lubricant is at least one selected from the group consisting of talc, and magnesium stearate; and the complexing agent is at least one selected from the group consisting of EDTA, BAL, and DMSA. More preferably, the disintegrant is microcrystalline cellulose, the flow aid is colloidal silica, the lubricant is magnesium stearate, and the complexing agent is EDTA. Preferably, the oral preparation is an enteric-coated tablet or an enteric-coated capsule. More preferably, the enteric-coated tablet is an enteric-coated sustained-release tablet, and the enteric-coated capsule is an enteric-coated sustained-release capsule.

The present disclosure provides a transdermal preparation, based on a total weight of the transdermal preparation, comprising: 20-65wt% of the aforementioned composition; 0.5-30wt% of a transdermal enhancer; 0.5-2wt5% of a gelling agent; and 1-30wt% of a solvent. Preferably, the transdermal enhancer is at least one selected from the group consisting of laurocapram, borneol, oleic acid, and peppermint oil; the gelling agent is at least one selected from the group consisting of carbomer, sodium hydroxypropylcellulose, sodium ethylcellulose, magnesium stearate, glycerol and polyethylene glycol; and the solvent is at least one selected from the group consisting of water, methanol, ethanol and DMSO.

The present disclosure provides a method for preparing the aforementioned composition, the method is carried out under a protection of an inert gas, and comprises the following steps:
(1) synthesis of component (B):
   (1-1) adding and fully dissolving an alkaline compound, an amino acid and glucose in solvent A, and allowing to react to obtain a reaction product of (1-1);
   (1-2) adding sodium borohydride to the reaction product of (1-1), allowing to react, then conducting acidification and purification to obtain a reaction product of (1-2);
   (1-3) adding and fully dissolving an alkaline compound and the reaction product of (1-2) in solvent A, and allowing to react to obtain component (B);
(2) synthesis of component (A):
   (2-1) dissolving an alkaline compound and the reaction product of (1-2) in water to obtain solution A;
   (2-2) dissolving CS₂ in solvent B to obtain solution B;
   (2-3) mixing solution A and solution B, allowing to react to obtain a reaction mixture, filtering the reaction mixture to obtain a filtrate, extracting the filtrate with solvent C to obtain a aqueous phase, and lyophilizing the aqueous phase to obtain a product;

   wherein a mixing ratio of solution A and solution B is a ratio which makes the product consist of only component (A);
   wherein solvent A is at least one selected from the group consisting of methanol, ethanol, acetone, acetonitrile, tetrahydrofuran and water; solvent B at least one selected from the group consisting of acetone, acetonitrile, tetrahydrofuran, dioxane and DMF; and solvent C is at least one selected from the group consisting of dichloromethane, ethyl acetate, propyl acetate, butyl acetate and isopropyl acetate; and
(3) mixing component (A), component (B) and an alkaline compound in proportion to obtain the composition.

The present disclosure provides another method for preparing the aforementioned composition, the method is carried out under a protection of an inert gas, and comprises the following steps:
(1) synthesis of component (B):
   (1-1) adding and fully dissolving an alkaline compound, an amino acid and glucose in solvent A, and allowing to react to obtain a reaction product of (1-1);
   (1-2) adding sodium borohydride to the reaction product of (1-1), allowing to react, then conducting acidification and purification to obtain a reaction product of (1-2);
   (1-3) adding and fully dissolving an alkaline compound and the reaction product of (1-2) in solvent A, and allowing to react to obtain component (B);
(2) synthesis of the composition:
   (2-1) dissolving an alkaline compound and component (B) in water to obtain solution A;
   (2-2) dissolving CS₂ in solvent B to obtain solution B; and
   (2-3) mixing solution A and solution B, allowing to react to obtain a reaction mixture, filtering the reaction mixture to obtain a filtrate, extracting the filtrate with solvent C to obtain a aqueous phase, and lyophilizing the aqueous phase to obtain a product;
wherein a mixing ratio of solution A and solution B is a ratio which makes the product be the composition.

Preferably, in step (2), a molar ratio of the alkaline compound to component (B) is 1.05: 1 to 1.5: 1, and a molar ratio of CS₂ to component (B) is 1.3: 1 to 5: 1. More preferably, the molar ratio of the alkaline compound to component (B) is 1.05: 1 to 1.3: 1, and the molar ratio of CS₂ to component (B) is 1.8: 1 to 3: 1.

Solvent A is at least one selected from the group consisting of methanol, ethanol, acetone, acetonitrile, tetrahydrofuran and water; solvent B at least one selected from the group consisting of acetone, acetonitrile, tetrahydrofuran, dioxane and DMF; and solvent C is at least one selected from the group consisting of dichloromethane, ethyl acetate, propyl acetate, butyl acetate and isopropyl acetate.

The present disclosure has the following beneficial technical effects: the composition obtained by the present disclosure has significantly improved stability compared to existing GDTC drugs, while maintaining excellent heavy metal-removing efficiency; due to the improved stability, the dosage can be reduced to about 1/4 of the original dosage without change of efficacy, thus improving the safety of drug use; meanwhile, the improved stability of the drug can effectively reduce production, transportation, and storage costs, extend the validity period, and effectively reduce costs of drugs.

### BRIEF DESCRIPTION OF THE DRAWINGS

FIG. 1 is a synthesis roadmap of the composition of the present disclosure;
FIG. 2 shows the daily copper excretion in rats of different groups in Example 26;
FIG. 3 shows the total daily bismuth excretion in urine of rats of different groups in Example 30;
FIG. 4 shows the ABR threshold shift result of the same stimulus frequency in mice before and after administration in Example 31;
FIG. 5 shows the tumors volume in different groups in Example 35; and
FIG. 6 shows the cell inhibitory effect of drugs of different groups on U87 malignant glioma in Example 36.

### DETAILED DESCRIPTION

The following is a clear and complete description of the technical solutions of the present disclosure through embodiments, in conjunction with the accompanying drawings in the specification. Obviously, the described embodiments are only partial embodiments of the present, disclosure, not all embodiments. Based on the embodiments of the present disclosure, all other embodiments obtained by ordinary technical personnel in this field without creative labor, belong to the scope of protection of the present disclosure.

### Synthesis of compositions

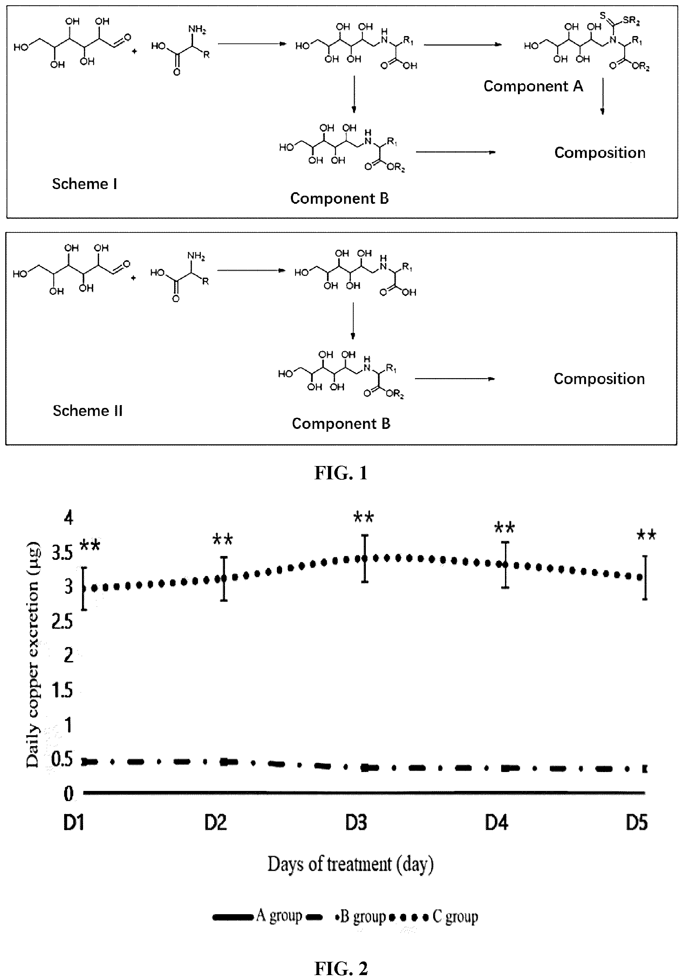

The synthetic route of the composition is shown in Figure 1.

### Scheme I

(1) Synthesis of component (B)
   (1-1) Adding and fully dissolving an alkaline compound, an amino acid and glucose in solvent A, and allowing to react to obtain a reaction product of (1-1).
   (1-2) Adding water dropwise to the reaction product of (1-1), controlling the temperature to 0-15 °C and adding sodium borohydride in batches; when the temperature is too high, the reaction would be too violent and out of control. Raising the temperature to 20-40°C and allow to react, cooling down to 0-15°C, and adding solvent A; when the temperature is too high, the reaction would be out of control, and a large amount of reaction liquid would overflow. Then conducting acidification and purification to obtain a reaction product of (1-2): diluting a resulting reaction system with water, adding concentrated hydrochloric acid dropwise to adjust pH to 1-5; no products will precipitate if the pH is too high or too low.
   (1-3) Adding an alkaline compound and the reaction product of (1-2) in solvent A to obtain component (B);
(2) Synthesis of component (A):
   (2-1) Dissolving an alkaline compound and the reaction product of (1-2) in water to obtain solution A;
   (2-2) Dissolving CS₂ in solvent B to obtain solution B;
   (2-3) Adding solution A dropwise to solution B. Stirring after the addition. Then conducting extration at 0-15°C, and lyophilizing to obtain component (A).

Component A), component B) and an alkaline compound are mixed in proportion to obtain the composition. The specific selection of Component A), component B), and the alkaline compound can be adjusted, and the cations in the composition can be composed of the same or different types of cations.

### Scheme II

(1) Synthesis of component (B)
   (1-1) Adding and fully dissolving an alkaline compound, an amino acid and glucose in solvent A, and allowing to react to obtain a reaction product of (1-1).
   (1-2) Adding water dropwise to the reaction product of (1-1), controlling the temperature to 0-15 °C and adding sodium borohydride in batches; when the temperature is too high, the reaction would be too violent and out of control. Raising the temperature to 20-40 °C and allowing to react, cooling down to 0-15 °C, and adding solvent A; when the temperature is too high, the reaction would be out of control, and a large amount of reaction liquid would overflow. Then conducting acidification and purification to obtain a reaction product of (1-2): diluting a resulting reaction system with water, adding concentrated hydrochloric acid dropwise to adjust pH to 1-5; no products will precipitate if the pH is too high or too low.
   (1-3) Adding an alkaline compound and the reaction product of (1-2) in solvent A to obtain component (B).
(2) Synthesis of the composition
   (2-1) Dissolving an alkaline compound and component (B) in water to obtain solution A; where the molar amount of the alkaline compound is 1.05~1.2 times that of component (B).
   (2-2) Dissolving CS₂ in solvent B to obtain solution B.
   (2-3) Adding solution A dropwise to solution B. Then conducting extraction at 0-15°C, and lyophilizing to obtain the composition.

By controlling the amount of added alkaline compounds and reaction time during the reaction process, the proportion of each component in the composition can be controlled; the cations in the composition are composed of the same type of cations.

### Embodiments

The embodiments of the present disclosure are described as follows. The embodiments described below are exemplary and are intended only to explain the present disclosure, and cannot be understood as limitations on the present disclosure. If the specific technology or conditions are not indicated in the embodiments, it shall be carried out in accordance with the techniques or conditions described in the literature in the art or in accordance with the product specification. The reagents or instruments used, where the manufacturer is not indicated, are conventional products commonly used in the field and available through market purchase. The content of each component in the embodiments of the present application is calculated by the mass without crystallization water unless otherwise specified.

The key raw materials used in the preparation process of the compositions and compounds represented by formula (1) and formula (2) are shown in Table 2.

**Table 2: Key raw materials and compounds represented by formula (1) and formula (2)**

| S N | Raw material | Formula of raw material | Formula (1) | Formula (2) |
|---|---|---|---|---|
| 1 | β-fluoro-alanine | 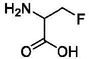 | 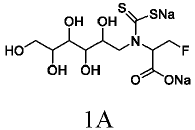 | 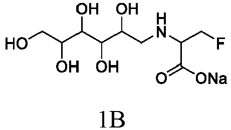 |
| 2 | valine | 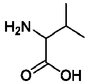 | 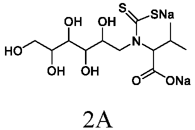 | 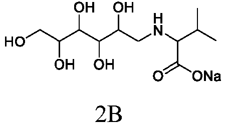 |
| 3 | methionine | 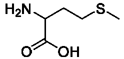 | 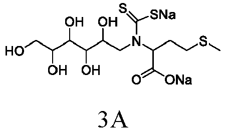 | 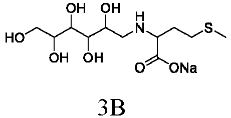 |
| 4 | S-(hydroxymethyl)-homocysteine | 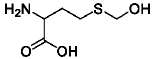 | 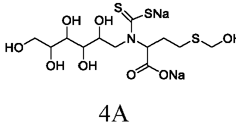 | 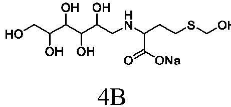 |
| 5 | glutamic acid | 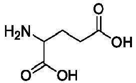 | 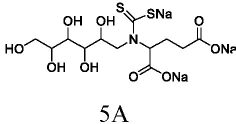 | 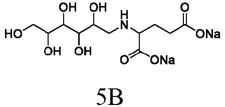 |
| 6 | cyclohexylalanine | 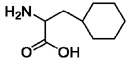 | 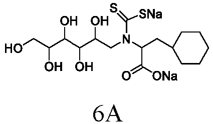 | 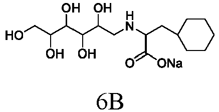 |
| 7 | 4-methoxyphenylalani ne | 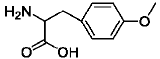 | 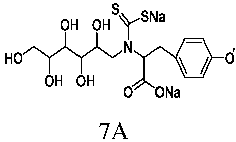 | 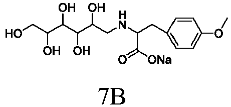 |
| 8 | 3-(2-thiophenyl) - alanine | 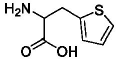 | 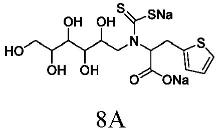 | 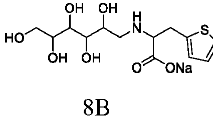 |
| 9 | 2-amino-3 -(thiazol-5-yl) propionic acid | 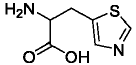 | 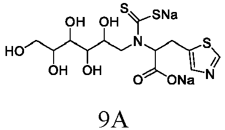 | 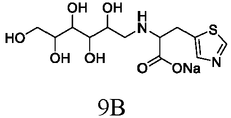 |
| 10 | serine | 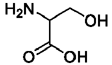 | 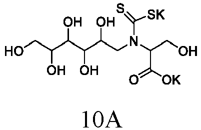 | 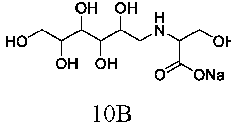 |
| 11 | S-allyl-cysteine | 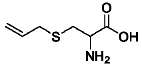 | 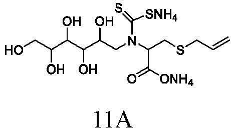 | 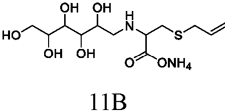 |

### Example 1

This embodiment involves the preparation of composition 3 in Table 1, which was carried out under inert gas protection. The specific steps were as follows.

### (1) Synthesis of compound 3B

(1-1) 48g of sodium hydroxide, 149g of methionine and 200g of glucose were added to 500mL of methanol at 10°C, stirred until clear, and a resulting mixture was heat up to 30°C for reaction for 10 h.

(1-2) 1000mL of water was added dropwise, the temperature was controlled at 5°C and 1100g of sodium borohydride was added in 5 batches, and the reaction was conducted for 12 hours. After heating to 35°C and reacting for 24 hours, the temperature was controlled at 10°C and 200mL of solvent (methanol) was added dropwise, a resulting mixture was stirred for 2 hours; the temperature was decreased to 4°C, 500mL of water was added, and 200mL of concentrated hydrochloric acid was added dropwise; crystallization, pulping, recrystallization, and harvesting were conducted to obtain (2,3,4,5,6-pentahydroxyhexyl) methionine with a purity greater than 99.6%.

(1-3) Sodium hydroxide was dissolved in methanol, equimolar (2,3,4,5,6-pentahydroxyhexyl) methionine was added and stirred until clear; filtration, spin dry, and recrystallization were conducted to obtain compound 3B with a purity greater than 99.9%.

The hydrogen spectra and carbon spectra of compound 3B determined by Bruker AV500MHz NMR spectrometer are as follows.
Ms (ES+,[M+H]⁺) 336
¹H NMR (500 MHz, D₂O) δ (ppm) 4.02 (m, 1H), 3.81-3.61 (m, 4H), 3.46 (t, 1H), 3.01~3.12 (m, 2H), 2.26 (m, 2H), 2.06~ 2.03 (m, 2H).2.02(s, 3H),
¹³C NMR (125 MHz, D2O) δ (ppm)168.83, 72.20, 71.07, 70.22, 70.71, 64.76, 60.15, 49.43, 30.05, 29.10, 14.94.

### (2) Synthesis of compound 3A

(2-1) 314g of (2,3,4,5,6-pentahydroxyhexyl) methionine and 216g of sodium carbonate were dissolved in 1500mL of purified water to obtain solution A, and the temperature was controlled at 20°C for use.

(2-2) 100mL of CS₂ was dissolved in 300mL of methanol to obtain solution B.

(2-3) Solution A was added dropwise to solution B. After the addition, a resulting mixture was stirred for 8 hours. 20 mL of CS₂/methanol solution was added to the reaction system. After cooling the system to 5°C and holding the temperature for 1 hour, filtration, 5 time of extraction with dichloromethane, and lyophilization were conducted to obtain compound 3A with a purity greater than 99%.

The hydrogen spectra and carbon spectra of compound 3A determined by Bruker AV500MHz NMR spectrometer are as follows.
Ms (ES+,[M+Na]⁺) 456
¹H NMR (500 MHz, D₂O) δ (ppm) 4.40 (t, 1H),3.96 (m, 1H), 3.79 (m, 1H), 3.56-3.59 (m,2H), 3.47 (m, 3H), 2.42~2.29 (m, 2H), 2.10 (m, 1H), 2.06 (s, 3H),1.87(m, 1H),
¹³C NMR (125 MHz, D₂O) δ (ppm) 208.23, 175.53, 73.12, 72.95, 71.31, 70.36, 66.76, 63.44, 52.88, 31.70, 30.20, 15.19.

### (3) Preparation of composition 3

800g of 3A, 150g of 3B, and 50g of ammonium bicarbonate were dissolved in 10L of water, and then lyophilized to obtain composition 3.

### Example 2

This embodiment involves the preparation of composition 5 in Table 1, which was carried out under inert gas protection. The specific steps were as follows.

### (1) Synthesis of compound 5B

(1-1) 98g of sodium hydroxide, 147g of glutamic acid and 200g of glucose were added to 1000mL of methanol at 20°C, stirred until clear, and a resulting mixture was heat up to 30°C for reaction for 10 h.

(1-2) 1000mL of water was added dropwise, the temperature was controlled at 8°C and 80g of sodium borohydride was added in 5 batches, and the reaction was conducted for 12 hours; after heating to 40°C and reacting for 10 hours, the temperature was decreased to 4°C, 500mL of water was added, and 200 mL of concentrated hydrochloric acid was added dropwise; crystallization, pulping, recrystallization, and harvesting were conducted to obtain the intermediate (2,3,4,5,6-pentahydroxyhexyl) glutamic acid with a purity greater than 99.4%.

(1-3) Sodium hydroxide was dissolved in methanol, equimolar (2,3,4,5,6-pentahydroxyhexyl) glutamic acid was added and stirred until clear; filtration, spin dry, and recrystallization were conducted to obtain compound 5B with a purity greater than 99.9%.

The hydrogen spectra and carbon spectra of compound 5B determined by Bruker AV500MHz NMR spectrometer are as follows.
Ms (ES+,[M+H]⁺) 356
¹H NMR (500 MHz, D₂O) δ (ppm) 3.91~3.78 (m, 3H), 3.72~3.48 (m, 4H), 3.08~2.83 (m, 2H), 2.60~2.40 (m, 2H), 2.12~1.81 (m, 2H).
¹³C NMR (125 MHz, D₂O) δ (ppm) 176.98, 176.76, 72.20, 72.07, 71.22, 70.71, 64.76, 59.81, 49.44, 31.29, 24.16.

### (2) Preparation of composition 5

(2-1) 800g of 5B and 300g of sodium carbonate were dissolved in 2500mL of purified water to obtain solution A, and the temperature was controlled at 30°C for use.

(2-2) 95mL of CS₂ was dissolved in 300mL of dioxane to obtain solution B.

(2-3) Solution A was added dropwise to solution B. After the addition, a resulting mixture was stirred for 8 hours. 1000mL of dioxane was further added, then filtration, 5 time of extraction with butyl acetate, and lyophilization of aqueous layer were conducted to obtain composition 5.

### Example 3

This embodiment involves the preparation of composition 7 in Table 1, which was carried out under inert gas protection. The specific steps were as follows.

### (1) Synthesis of compound 7B

(1-1) 46g of sodium hydroxide, 195g of 4-methoxyphenylalanine, and 220g of glucose were added to 600mL of water at 20°C, stirred until clear, and a resulting mixture was heat up to 35°C for reaction for 20h.

(1-2) 800mL of water was added dropwise, the temperature was controlled at 6°C and 120g of sodium borohydride was added in 5 batches, and the reaction was conducted for 12 hours; after heating to 42°C and reacting for 10 hours, 300mL of solvent acetonitrile was added dropwise, a resulting mixture was stirred for 2 hours; 250mL of concentrated hydrochloric acid was added dropwise; the temperature was decreased to room temperature, crystallization, pulping, recrystallization, and harvesting were conducted to obtain an intermediate with a purity greater than 99.8%.

(1-3) Sodium carbonate was dissolved in methanol, equimolar intermediate was added and stirred until the reaction was completed; filtration, spin dry, and recrystallization were conducted to obtain compound 7B with a purity greater than 99.9%.

The hydrogen spectra and carbon spectra of compound 7B determined by Bruker AV500MHz NMR spectrometer are as follows.
Ms (ES+,[M+H]⁺) 382
¹H NMR (500 MHz, CD₃OD) δ (ppm) 7.12 (m,2H), 6.80(m, 2H), 3.93 (dt, 1H), 3.86 (dd, J = 8.5, 7.5 Hz, 1H), 3.78 (s, 3H), 3.78~3.75 (m, 1H), 3.72~3.61 (m, 4H), 2.97~2.83 (m, 4H).
¹³C NMR (125 MHz,CD₃OD) δ (ppm) 171.24, 158.51, 131.11, 130.27, 113.64, 73.20, 73.07, 72.22, 71.71, 64.76, 62.47, 55.33, 49.39, 36.42.

### (2) Synthesis of compound 7A

(2-1) 314g of intermediate and 350g of sodium carbonate were dissolved in 1500mL of purified water to obtain solution A, and the temperature was controlled at 30°C for use.

(2-2) 100mL of CS₂ was dissolved in 300mL of methanol to obtain solution B.

(2-3) Solution A was added dropwise to solution B. After the addition, a resulting system was stirred for 8 hours. After cooling the system to 5°C and holding the temperature for 1 hour, filtration, 5 time of extraction with ethyl acetate, and lyophilization of aqueous layer were conducted to obtain compound 7A with a purity greater than 99%.

The hydrogen spectra and carbon spectra of compound 7A determined by Bruker AV500MHz NMR spectrometer are as follows.
Ms (ES+,[M+H]⁺) 480
¹H NMR (500 MHz, D₂O) δ (ppm) 7.08 (m,2H), 6.85 (m, 2H), 4.48 (t, 1H), 4.14~3.91 (m, 3H), 3.83~3.72 (m, 4H), 3.70~3.59 (m, 3H), 3.16~3.03 (m, 2H).
¹³C NMR (125 MHz, D₂O) δ (ppm) 204.05, 171.96, 153.51, 134.04, 127.90, 113.64, 73.12, 72.95, 72.31, 70.48, 65.76, 64.61, 55.33, 53.06, 33.03.

### (3) Preparation of composition 7

900g of 7A, 85g of 7B, and 15g of sodium bicarbonate were dissolved in 10L of water, and then lyophilized to obtain composition 7.

### Example 4

This embodiment involves the preparation of composition 10 in Table 1, which was carried out under inert gas protection. The specific steps were as follows.

### (1) Synthesis of compound 10B

(1-1) 60g of potassium hydroxide, 105g of serine and 200g of glucose were added to 600mL of water at 20°C, stirred until clear, and a resulting mixture was heat up to 35 °C for reaction for 10h.

(1-2) 800mL of water was added dropwise, the temperature was controlled at 6°C and 180g of sodium borohydride was added in 5 batches, and the reaction was conducted for 12 hours; after heating to 42°C and reacting for 18 hours, the temperature was controlled at 10°C and 280mL of solvent tetrahydrofuran was added dropwise, a resulting mixture was stirred for 2 hours; 250 mL of concentrated hydrochloric acid was added dropwise; crystallization, pulping, recrystallization, and harvesting were conducted to obtain an intermediate with a purity greater than 99.8%.

(1-3) Sodium hydroxide was dissolved in acetonitrile, equimolar intermediate was added and stirred until reaction was completed; filtration, spin dry, and recrystallization were conducted to obtain compound 10B with a purity greater than 99.9%.

The hydrogen spectra and carbon spectra of compound 10B determined by Bruker AV500MHz NMR spectrometer are as follows.
Ms (ES+, [M+H]⁺) 292;
¹H NMR (500 MHz, D₂O) δ (ppm) 3.86~3.72 (m, 4H), 3.71~3.54 (m, 5H), 2.97~2.85 (m, 2H).
¹³C NMR (125 MHz, D₂O) δ (ppm) 170.57, 73.20, 73.07, 72.22, 71.77, 64.76, 61.95, 61.95, 49.47.

### (2) Synthesis of compound 10A

(2-1) 314g of intermediate and 350g of potassium carbonate were dissolved in 1500mL of purified water to obtain solution A, and the temperature was controlled at 30°C for use.

(2-2) 100mL of CS₂ was dissolved in 300mL of dioxane to obtain solution B.

(2-3) Solution A was added dropwise to solution B. After the addition, a resulting system was stirred for 8 hours. After cooling the system to 5°C and holding the temperature for 1 hour, filtration, 8 time of extraction with ethyl acetate, and lyophilization of aqueous layer were conducted to obtain compound 10A with a purity greater than 99%.

The hydrogen spectra and carbon spectra of compound 10A determined by Bruker AV500MHz NMR spectrometer are as follows.
Ms (ES+,[M+K]⁺) 460;
¹H NMR (500 MHz, D₂O) δ (ppm) 4.61 (t, 1H), 4.15~4.06 (m, 2H), 4.03~3.93 (m, 3H), 3.89~3.75 (m, 2H), 3.68~3.50 (m, 3H).
¹³C NMR (125 MHz, D₂O) δ (ppm) 204.34, 176.56, 73.12, 73.01, 72.31, 70.45, 64.76, 64.20, 61.16, 52.54.

### (3) Preparation of composition 10

900g of 10A, 90g of 10B, and 10g of potassium bicarbonate were dissolved in 10L of water, and then lyophilized to obtain composition 10.

### Example 5

This embodiment involves the preparation of composition 11 in Table 1, which was carried out under inert gas protection. The specific steps were as follows.

### (1) Synthesis of compound 11B

(1-1) 48g of sodium hydroxide, 161g of S-allyl-cysteine and 210g of glucose were added to 200mL of tetrahydrofuran at 30°C, stirred until clear, and reacted at 30°C for 10h.

(1-2) 1000mL of water was added dropwise, the temperature was controlled at 10°C and 100g of sodium borohydride was added in 5 batches, and the reaction was conducted for 12 hours; after heating to 30°C and reacting for 10 hours, the temperature was controlled at 10°C and 200mL of solvent tetrahydrofuran was added dropwise, a resulting mixture was stirred for 2 hours; the temperature was decreased to 10°C, 500mL of water was added, and 240mL of concentrated hydrochloric acid was added dropwise; crystallization, pulping, recrystallization, and harvesting were conducted to obtain an intermediate with a purity greater than 99.2%.

(1-3) An appropriate amount of intermediate was added to ammonia water, stirred until clear; filtration, spin dry, and recrystallization were conducted to obtain compound 11B with a purity greater than 99.9%.

The hydrogen spectra and carbon spectra of compound 11B determined by Bruker AV500MHz NMR spectrometer are as follows.
Ms (ES+,[M+H]⁺) 343;
¹H NMR (500 MHz, D₂O) δ (ppm) 5.80 (m, 1H), 5.11 (m, 2H), 3.85~3.52 (m, 7H), 3.24~3.14 (m, 2H), 3.03~2.85 (m, 4H).
¹³C NMR (125 MHz, D₂O) δ (ppm) 168.82, 133.38, 117.29, 73.20, 73.07, 72.22, 71.57, 64.76, 59.06, 49.65, 35.65, 33.52.

### (2) Synthesis of compound 11A

(2-1) 325g of intermediate and 60g of ammonium hydroxide were dissolved in 1600mL of purified water to obtain solution A, and the temperature was controlled at 30°C for use.

(2-2) 200mL of CS₂ was dissolved in 300 mL of methanol to obtain solution B.

(2-3) Solution A was added dropwise to solution B. After the addition, a resulting system was stirred for 8 hours. After cooling the system to 5°C and holding the temperature for 1 hour, filtration, 5 time of extraction with isopropyl acetate, and lyophilization of aqueous layer were conducted to obtain compound 11A with a purity greater than 99%.

The hydrogen spectra and carbon spectra of compound 11A determined by Bruker AV500MHz NMR spectrometer are as follows.
Ms (ES+,[M+H]⁺) 437;
¹H NMR (500 MHz, D₂O) δ (ppm) 5.80 (m, 1H), 5.11 (m, 2H), 4.68 (t, 1H), 4.16~3.98 (m, 3H), 3.89~3.75 (m, 2H), 3.67~3.51 (m, 3H), 3.27~3.07 (m, 4H).
¹³C NMR (125 MHz, D₂O) δ (ppm) 210.45, 170.18, 133.45, 117.25, 73.12, 73.01, 72.31, 70.31, 64.76, 61.31, 52.70, 35.82, 33.58.

### (3) Preparation of composition 11

910g of 11A, 80g of 11B, and 10g of sodium carbonate were dissolved in 10L of water, and then lyophilized to obtain composition 11.

### Example 6

The present embodiment involves the preparation of compositions 1, 2, 4, 6, 8, 9 in Table 1, steps (1) and steps (2) of the preparation process were the same as Example 1 excepted that methionine in Example 1 was replaced with the same moles of raw materials corresponding to compositions 1, 2, 4, 6, 8, 9 in Table 2.

### Steps (3) were as follows, respectively.

Composition 1: 860g of 1A, 132g of 1B, 8g of potassium carbonate were dissolved in 10L of water, and then lyophilized to obtain composition 1.

The hydrogen spectra and carbon spectra of compound 1A determined by Bruker AV500MHz NMR spectrometer are as follows.
Ms (ES+,[M+Na]⁺) 414;
¹H NMR (500 MHz, D₂O) δ (ppm) 5.00 (d, 1H), 4.90(d, 1H), 4.36 (m, 1H), 4.17 (m,1H), 4.01 (m, 1H), 3.95 (m, 1H), 3.90 (m,1H), 3.72 ~ 3.68 (m, 1H), 3.60 ~ 3.55(m, 1H), 3.50 ~ 3.42(m, 1H), 3.53 (q, 1H).
¹³C NMR (125 MHz, D₂O) δ (ppm) 213.73, 170.59, 81.10, 71.12, 71.01, 69.31, 69.53, 62.76, 61.14, 51.09.

The hydrogen spectra and carbon spectra of compound 1B determined by Bruker AV500MHz NMR spectrometer are as follows.
Ms (ES+,[M+H]⁺) 294;
¹H NMR (500 MHz, D₂O) δ (ppm) 4.55 (d,2H), 3.82 (t, 1H), 3.88(m, 3H), 3.53 (m, 1H), 3.38 (m, 2H), 2.63(t, 2H).
¹³C NMR (125 MHz, D₂O) 175.12, 82.47, 75.20, 74.07, 71.22, 70.76, 65.76, 64.97, 47.39

Composition 2: 900g of 2A, 90g of 2B, 10 g of ammonium hydroxide were dissolved in 10L of water, and then lyophilized to obtain composition 2.

The hydrogen spectra and carbon spectra of compound 2A determined by Bruker AV500MHz NMR spectrometer are as follows.
Ms (ES+,[M+Na]⁺) 424;
¹H NMR (500 MHz, D₂O) δ (ppm) 4.91 (d, 1H), 4.26 (m, 1H), 4.18(m, 1H), 4.04 ~ 3.93 (m, 2H), 3.80 ~ 3.73 (m, 1H), 3.71 ~ 3.58 (m, 3H), 2.76 ~ 2.63 (m, 1H), 0.96 (m, 6H).
¹³C NMR (125 MHz, D₂O) δ (ppm) 205.08, 173.50, 73.12, 72.95, 72.31, 70.47, 67.97, 64.76, 53.25, 29.26, 18.31.

The hydrogen spectra and carbon spectra of compound 2B determined by Bruker AV500MHz NMR spectrometer are as follows.
Ms (ES+,[M+Na]⁺) 326;
¹H NMR (500 MHz, D₂O) δ (ppm) 3.85 ~ 3.76 (m, 2H), 3.80 ~ 3.73 (m, 1H), 3.68 ~ 3.62 (m, 1H), 3.62 ~ 3.54 (m, 2H), 3.51 (d, J1H), 2.95 ~ 2.87 (m, 1H), 2.83 ~ 2.74 (m, 1H), 1.96 (m, 1H), 0.93 (d, 3H), 0.88 (d, 3H).
¹³C NMR (125 MHz, D₂O) δ (ppm) 174.20, 73.20, 73.07, 72.22, 71.70, 65.76, 64.76, 49.62, 29.61, 18.79.

Composition 4: 920g of 4A, 60g of 4B, 20g of dipotassium hydrogenphosphate were dissolved in 10L of water, and then lyophilized to obtain composition 4.

The hydrogen spectra and carbon spectra of compound 4A determined by Bruker AV500MHz NMR spectrometer are as follows.
Ms (ES+,[M+Na]⁺) 472;
¹H NMR (500 MHz, D₂O) δ (ppm) 4.32 (t,1H), 4.22~3.94 (m,5H), 3.88~3.75 (m,2H), 3.68~3.51 (m, 3H), 2.88~2.74 (m, 2H), 2.07 (m,2H).
¹³C NMR (125 MHz, D₂O) δ (ppm) 206.05, 173.65, 138.61, 127.33, 126.57, 125.74, 73.12, 72.95, 72.31, 70.48, 64.76, 63.59, 53.03, 32.10.

The hydrogen spectra and carbon spectra of compound 4B determined by Bruker AV500MHz NMR spectrometer are as follows.
Ms (ES+,[M+H]+) 352;
¹H NMR (500 MHz, D₂O) δ (ppm) 4.20 (d, 2H), 3.85~3.74 (m, 3H), 3.68~3.49 (m, 4H), 2.99~2.67 (m, 4H), 1.89 (m, 2H).
¹³C NMR (125 MHz, D₂O) δ (ppm) 171.15, 138.89, 126.86, 125.77, 73.20, 73.07, 72.22, 71.71, 64.76, 61.85, 49.39, 32.89.

Composition 6: 910g of 6A, 75g of 6B, 15g of sodium acetate were dissolved in 10L of water, and then lyophilized to obtain composition 6.

The hydrogen spectra and carbon spectra of compound 6A determined by Bruker AV500MHz NMR spectrometer are as follows.
Ms (ES+,[M+Na]⁺) 478;
¹H NMR (500 MHz, D₂O) δ (ppm) 4.18~4.06 (m, 3H), 3.63~3.44 (m, 3H), 3.29~3.01 (m, 3H), 2.10~1.83 (m, 3H), 1.59~1.15 (m, 10H)
¹³C NMR (125 MHz, D₂O) δ (ppm) 204.23, 171.80, 73.12, 72.95, 72.31, 70.36, 64.76, 62.10, 52.95, 34.38, 34.12, 32.76, 26.16, 25.99.

The hydrogen spectra and carbon spectra of compound 6B determined by Bruker AV500MHz NMR spectrometer are as follows.
Ms (ES+,[M+H]⁺) 358;
¹H NMR (500 MHz, D₂O) δ (ppm) 4.20 (d, 2H), 3.84 ~ 3.72 (m, 3H), 3.68 ~ 3.62 (m, 4H), 2.98 ~2 .68(m,3H), 1.89~1.56 (mm, 10H).
¹³C NMR (125 MHz, D₂O) δ (ppm) 172.04, 73.14, 72.22, 71.71, 64.76, 59.63, 49.51, 35.56, 33.61, 33.11, 26.16, 25.99.

Composition 8: 900g of 8A, 85g of 8B, 15g of sodium bicarbonate were dissolved in 10L of water, and then lyophilized to obtain composition 8.

The hydrogen spectra and carbon spectra of compound 8A determined by Bruker AV500MHz NMR spectrometer are as follows.
Ms (ES+,[M+Na]⁺) 478;
¹H NMR (500 MHz, D₂O) δ (ppm) 7.26 (m,1H), 6.92 (m,1H), 6.75 (m,1H), 4.64 (t,1H), 4.18~3.94 (m, 3H), 3.89~3.76 (m, 2H), 3.67~3.51 (m, 3H), 3.43~3.27 (m, 2H).
¹³C NMR (125 MHz, D₂O) δ (ppm) 211.05, 178.68, 152.88, 142.18, 133.53, 73.12, 72.95, 72.31, 70.48, 64.76, 64.29, 53.03, 28.99.

The hydrogen spectra and carbon spectra of compound 8B determined by Bruker AV500MHz NMR spectrometer are as follows.
Ms (ES+,[M+H]⁺) 358;
¹H NMR (500 MHz, D₂O) δ (ppm) 7.26 (d, 1H), 6.93 (d, 1H), 6.72 (dd, 1H), 3.86~3.56 (m, 7H), 3.13~2.87 (m, 4H).
¹³C NMR (125 MHz, D₂O) δ (ppm) 176.22, 152.92, 142.59, 133.81, 73.20, 73.07, 72.22, 71.70, 64.76, 61.48, 49.39, 30.68.

Composition 9: 910g of 9A, 85g of 9B, 5g of sodium bicarbonate were dissolved in 10 L water, and then lyophilized to obtain composition 9.

The hydrogen spectra and carbon spectra of compound 9A determined by Bruker AV500MHz NMR spectrometer are as follows.
Ms (ES+,[M+H]⁺) 479;
¹H NMR (500 MHz, D₂O) δ (ppm) 8.57 (d, 1H), 7.42 (d, 1H), 4.64 (t, 1H), 4.19~3.94 (m, 3H), 3.90~3.75 (m, 2H), 3.69~3.41 (m, 4H), 3.29 (m, 1H).
¹³C NMR (125 MHz, D₂O) δ (ppm) 206.05, 170.68, 152.88, 142.18, 133.53, 73.12, 72.95, 72.31, 70.48, 64.76, 64.29, 53.03, 28.99.

The hydrogen spectra and carbon spectra of compound 9B determined by Bruker AV500MHz NMR spectrometer are as follows.
Ms (ES+,[M+H]⁺) 359;
¹H NMR (500 MHz, D₂O) δ (ppm) 8.58 (d, 1H), 7.43 (d, 1H), 3.91~3.73 (m, 4H), 3.72~3.53 (m, 3H), 3.24~2.85 (m, 4H).
¹³C NMR (125 MHz, D₂O) δ (ppm) 171.22, 152.92, 142.59, 133.81, 73.20, 73.07, 72.22, 71.70, 64.76, 61.48, 49.39, 30.68.

### Example 7

This embodiment involves the preparation of powder injections with composition 3 prepared in Example 1 as the main component.

1250g of composition, 5g of ETDA, 400g of mannitol, and 400g of ammonium hydroxide were dissolved in 15 L of ice water in a nitrogen atmosphere, stirred evenly, and automatically divided into 20ml vials with 7.5mL per vial; pre-cooling to -40°Cand lyophilization were conducted to obtain composition 3-powder injections containing 500mg of composition 3A per vial.

### Example 8

This embodiment involves the preparation of powder injections with composition 5 prepared in Example 2 as the main component.

1111g of composition 5, 10g of ETDA, 300g of trehalose, and 50g of sodium bicarbonate were dissolved in 15L of ice water in a nitrogen atmosphere, stirred evenly, and automatically divided into 20ml vials with 7.5 mL per vial; pre-cooling to -40°Cand lyophilization were conducted to obtain composition 5-powder injections containing 500mg of composition 5A per vial.

### Example 9

This embodiment involves the preparation of powder injections with composition 7 prepared in Example 3 as the main component.

1100g of composition 7, 1g of BAL, 200g of mannitol, and 85g of ammonium acetate were dissolved in 15L of ice water in a nitrogen atmosphere, stirred evenly, and automatically divided into 20ml vials with 7.5 mL per vial; pre-cooling to -40°C and lyophilization were conducted to obtain composition 7-powder injections containing 500mg of composition 7A per vial.

### Example 10

The present embodiment involves the preparation of enteric-coated granules (tablets) with composition 3 prepared in Example 1 as the main component.

300g of composition, 1g of EDTA, 287g of microcrystalline cellulose (PH102), 6g of colloidal silica, 6g of magnesium stearate MF-2-V, 17g of film-coating premix (gastric-soluble), and 480g of film-coating premix (enteric-coated) were respectively sieved through 40 mesh sieve for pretreatment; then microcrystalline cellulose, composition, EDTA, colloidal silica, and magnesium stearate were sieved together through 40 mesh sieve, then mixed, and added to the dry granulator; particles of 14-30 mesh were collected, and isolation-coated in a fluidized bed with a gastric-soluble coating solution which was prepared at a concentration of 5%, then enteric-coated with an enteric-coating solution which was prepared at a concentration of 20% to obtain enteric-coated granules. Enteric-coated tablets were obtained by enteric-coating after tableting.

### Example 11

The present embodiment involves the preparation of enteric-coated capsules with composition 3 prepared in Example 1 as the main component.

300g of composition, 3g of EDTA, 285g of microcrystalline cellulose (PH102), 6g of colloidal silica, and 6g of magnesium stearate MF-2-V were respectively sieved through 40 mesh sieve for pretreatment; then microcrystalline cellulose, composition, EDTA, colloidal silica, and magnesium stearate were sieved together through 40 mesh sieve, then mixed, and added to the dry granulator; particles of 14-30 mesh were collected, and filled into No.2 enteric-coated capsules with 300mg/capsule to obtain enteric-coated capsules.

### Example 12

The present embodiment involves the preparation of enteric-coated sustained-release granules (tablets) with composition 3 prepared in Example 1 as the main component.

300g of composition, 6g of EDTA, 200g of microcrystalline cellulose (PH102), 84g of hypromellose, 5g of colloidal silica, 5g of magnesium stearate MF-2-V, 17g of film-coating premix (gastric-soluble), and 480g of film-coating premix (enteric-coated) were respectively sieved through 40 mesh sieve for pretreatment; then microcrystalline cellulose, composition, EDTA, colloidal silica, and magnesium stearate were sieved together through 40 mesh sieve, then mixed, and added to the dry granulator; particles of 14-30 mesh were collected, and isolation-coated in a fluidized bed with a gastric-soluble coating solution which was prepared at a concentration of 5%, then enteric-coated with an enteric-coating solution which was prepared at a concentration of 20% to obtain enteric-coated sustained-release granules. Enteric-coated sustained-release tablets were obtained by enteric-coating after tableting.

### Example 13

The present embodiment involves the preparation of enteric-coated sustained-release capsules with composition 3 prepared in Example 1 as the main component.

300g of composition, 5g of EDTA, 200g of microcrystalline cellulose (PH102), 88g of hypromellose, 6g of colloidal silica, and 6g of magnesium stearate MF-2-V were respectively sieved through 40 mesh sieve for pretreatment; then microcrystalline cellulose, composition, EDTA, colloidal silica, and magnesium stearate were sieved together through 40 mesh sieve, then mixed, and added to the dry granulator; particles of 14-30 mesh were collected, and filled into No.2 enteric-coated capsules with 300mg/capsule to obtain enteric-coated sustained-release capsules.

### Example 14

The present embodiment involves the preparation of transdermal preparations with composition 3 prepared in Example 1 as the main component.

200g of composition, 10g of EDTA, 80g of hydroxypropyl cellulose sodium, 100g of laurocapram, 200g of peppermint oil, 80g of magnesium stearate, 40g of polyethylene glycol 400, and 290g of water were mixed and stirred into a gel, evenly smeared on a backing material with an area of 10000cm², and divided according to a needed size, thus transdermal preparations with composition 3 as the main component was obtained.

### Example 15

The solid stability and compatibility stability of composition 3A, composition 3 prepared in Example 1, composition 3-1 prepared by 80wt% 3A and 20wt% 3B, composition 3-2 prepared by 80wt% 3A and 20wt% sodium carbonate, and the powder injection prepared in Example 7 with composition 3 as the main component (hereinafter referred to as "composition 3-preparation") were studied in this embodiment.

Compound 3A in the present disclosure, that is, D3 in Chinese patent 2005100353771, that is, GMDTC stated in the background, is referred to as "D3" in the present embodiment and subsequent embodiments, for comparison with Chinese patent 2005100353771.

D3 and composition 3-1, composition 3-2, composition 3, composition 3-preparation were placed at -25°C, 5 ± 3°C, 25 ± 2°C/60 ± 5%RH, 40 ± 2°C/75 ± 5%RH environment for 0d, 5d, 10d, 30d and 180d (d refers to day), respectively; changes of the main component content (the main component content of 0d was converted to 100%) of each group were measured, as shown in Table 3.

**Table 3: Changes of the main component content over time in different groups under different conditions**

| Condition | Group | 0d | 5d | 10d | 30d | 180d |
|---|---|---|---|---|---|---|
| 40±2°C/ | D3 | 100% | 91.2% | 84.3% | 60.3% | / |
| 75±5%RH | composition 3-1 | 100% | 93.1% | 87.3% | 73.4% | / |
| | composition 3-2 | 100% | 92.4% | 88.5% | 81.7% | / |
| | composition 3 | 100% | 96.4% | 95.4% | 91.2% | / |
| | composition 3-preparation | 100% | 99.0% | 98.3% | 97.5% | / |
| 25±2°C/ 60±5%RH | D3 | 100% | 95.1% | 90.3% | 84.6% | 64.2% |
| | composition 3-1 | 100% | 96.8% | 93.4% | 88.5% | 83.5% |
| | composition 3-2 | 100% | 97.7% | 94.1% | 87.7% | 82.3% |
| | composition 3 | 100% | 99.4% | 97.3% | 95.5% | 94.2% |
| | composition 3-preparation | 100% | 101.1% | 99.1% | 98.9% | 98.3% |
| 5±3°C | D3 | 100% | 97.3% | 96.5% | 90.8% | 81.2% |
| | composition 3-1 | 100% | 97.8% | 96.5% | 93.3% | 90.5% |
| | composition 3-2 | 100% | 98.2% | 97.8% | 94.7% | 91.8% |
| | composition 3 | 100% | 100.4% | 99.7% | 98.9% | 99.2% |
| | composition 3-preparation | 100% | 99.9% | 100.2% | 101.5% | 100.5% |
| -25°C | D3 | 100% | 101.2% | 99.8% | 100.7% | 99.9% |
| | composition 3-1 | 100% | 100.4% | 99.7% | 99.5% | 100.1% |
| | composition 3-2 | 100% | 99.7% | 99.6% | 100.0% | 99.3% |
| | composition 3 | 100% | 100.3% | 101.2% | 100.4% | 100.3% |
| | composition 3-preparation | 100% | 101.5% | 100.3% | 100.6% | 101.2% |

The data more than 100% in the table were due to integration errors (the same below).

D3, composition 3-1, composition 3-2, composition 3 and composition 3-preparation were respectively prepared into a solution with the main component concentration being 2mg/ml, with normal saline and 5% glucose injection as the solvent; pH was detected, and changes of the main component content (the main component content of 0d was converted to 100%) of each group were measured after being placed at room temperature for 8 hours, as shown in Table 4.

**Table 4: Changes of the main component content of different groups in solution over time**

| Solvent | Group | pH | 0h | 1h | 2h | 4h | 8h |
|---|---|---|---|---|---|---|---|
| Normal saline | D3 | 9.7 | 100% | 95% | 83% | 73% | 71% |
| | composition 3-1 | 9.6 | 100% | 97% | 92% | 87% | 83% |
| | composition 3-2 | 10.5 | 100% | 99% | 93% | 89% | 85% |
| | composition 3 | 9.7 | 100% | 101% | 99% | 99% | 97% |
| | composition 3-preparation | 9.7 | 100% | 99% | 100% | 101% | 98% |
| 5% glucose injection | D3 | 9.7 | 100% | 90% | 81% | 69% | 54% |
| | composition 3-1 | 9.6 | 100% | 95% | 90% | 88% | 85% |
| | composition 3-2 | 10.5 | 100% | 94% | 90% | 88% | 87% |
| | composition 3 | 9.7 | 100% | 100% | 98% | 98% | 95% |
| | composition 3-preparation | 9.7 | 100% | 101% | 100% | 101% | 99% |

It can be seen from Table 3 that composition 3 and composition 3-preparation are stable for 180d at 5 ± 3°C; the main component content of composition 3-1 and composition 3-2 under this condition decreased by about 9% on 180d, while the main component content of D3 decreased by about 18.8% on 180d; when the temperature rose to 25 2 °C, stability of composition 3-1 and composition 3-2 decreased, with the the main component content decreasing by about 17% on 180d, while the main component of composition 3 decreased by 5.8%, and composition 3-preparation was relatively stable. It can be seen that stability of composition 3-preparation and composition 3 were significantly better than that of composition 3-1, composition 3-2 and D3, which can effectively reduce transportation and storage costs and extend the validity period.

It can be seen from Table 4 that the stability of composition 3-preparation and composition 3 in the test stage were relatively good (basically unchanged), while the stability of D3 was poor, with a hydrolysis of 30% ~ 50%, after 8 hours; composition 3-1, composition 3-2 had a hydrolysis of 10% ~ 20. It can be seen that the compatibility stability of composition 3-preparation and composition 3 are significantly better than that of composition 3-1, composition 3-2 and D3, and can be used in clinical practice.

### Example 16

The acute toxicity of composition 3 prepared in Example 1, composition 3-powder injection prepared in Example 7, the oral preparations of composition 3 prepared in Examples 10, 11, 13 were studied in this embodiment.

SPF-grade SD rats, 180~220 g, 70 males and 70 females, were divided into 7 groups, 10 males and 10 females in each group. Rats in group 1 were intravenous injected with 3g/kg of D3; rats in group 2 were intravenous injected with 3g/kg of composition 3; rats in group 3 were intravenous injected with 3g/kg of composition 3-powder injection; rats in group 4 were orally administrated with 5g/kg of composition 3-enteric-coated granule; rats in group 5 were orally administrated with 5g/kg of composition 3-enteric-coated capsule; rats in group 6 were orally administrated with 5g/kg of composition 3-enteric-coated sustained-release capsule; group 7 was a blank group. After administration, the poisoning situation was observed every day, and no obvious poisoning reaction was found. The weight of the animals was measured on days 0, 3, 7 and 14, and the results showed that the animal weight was normal, there was no obvious toxicity, and there was no difference in the organs after dissection. Therefore, when D3 is injected, rat MTD is greater than 3g/kg; When taken orally, rat MTD is greater than 5g/kg; the substance in each group has a extremely low toxicity, with a toxicity comparable to that of D3.

Ordinary grade beagles 8~10 kg, 70 males and 70 females, were divided into 7 groups, 10 males and 10 females in each group. Beagles in group 1 were intravenous injected with 2g/kg of D3; beagles in group 2 were intravenous injected with 2g/kg of composition 3; beagles in group 3 were intravenous injected with 2g/kg of composition 3-powder injection; beagles in group 4 were orally administrated with 5g/kg of composition 3-enteric-coated granule; beagles in group 5 were orally administrated with 5g/kg of composition 3-enteric-coated capsule; beagles in group 6 were orally administrated with 5g/kg of composition 3-enteric-coated sustained-release capsule; group 7 was a blank group. After administration, the poisoning situation was observed every day, and no obvious poisoning reaction was found. The weight of the animals was measured on days 0, 3, 7 and 14, and the results showed that the animal weight was normal, there was no obvious toxicity. Therefore, when D3 is injected, dog MTD is greater than 2g/kg; When taken orally, dog MTD is greater than 5g/kg; the substance in each group has a extremely low toxicity, with a toxicity comparable to that of D3.

### Example 17

The long-term toxicity of composition 3 prepared in Example 1 and composition 3-powder injection prepared in Example 7 were studied in this embodiment.

SPF grade SD rats, 180~220g, 105 males and 105 females, were divided into 7 groups, 15 males and 15 females in each group. Rats in group 1 were intravenous injected with 200mg/kg of composition 3-powder injection; rats in group 2 were intravenous injected with 500mg/kg of composition 3-powder injection; rats in group 3 were intravenous injected with 1000mg/kg of composition 3-powder injection; the administration lasted for 28 days. Rats in group 5 were intravenous injected with 200mg/kg of composition 3; rats in group 6 were intravenous injected with 500mg/kg of composition 3; rats in group 7 were intravenous inj ected with 1000mg/kg of composition 3; the administration lasted for 28 days. Group 4 was a blank group. The poisoning situation was observed every day, blood biochemistry was detected after the end of administration; there was no obvious difference between composition 3 and composition 3-preparation; in the high-dose groups of both composition 3 and composition 3-preparation, there was a slight decrease in HGB, as well as slight redness and ulceration at the tail injection site. The animal weight was measured on days 0, 7, 14, 21 and 28, and the results showed that the animal weight was normal. 10 males and females in each group were sacrificed 24h after 28 days of administration, and dissected; all organs were observed to be generally abnormal, all the organ coefficients were normal, and there were no abnormalities in all organs after microscopic examination. The remaining 5 males and 5 females in each group recovered for 28 days, then blood biochemistry was detected; various functions were normal; after dissection, all organs were generally observed, there were no abnormalities, and all the organ coefficients were normal; there were no abnormalities in all organs after microscopic examination. Rat NOAEL values of composition 3 and composition 3-powder injection are both 500mg/kg.

### Example 18

The cadmium-removing effects of D3, compound 3B, composition 3 prepared in Example 1, composition 3-powder injection prepared in Example 7 (referred to as composition 3-preparation) were studied in this embodiment.

205 male New Zealand rabbits, 1.8~2.2 kg, were randomly divided into a blank control group with 25 rabbits and chronic cadmium poisoning groups with a total of 180 rabbits. The model rabbits were ear margin intravenous injected with a mixed solution containing 1.5µmol/kg CdCl₂ and 30µmol/kg thioethanol (ME) according to weight, once/d for continuous 5 days, and observed for 35 days. After 35 days, qualified rabbits were selected according to weight, including 18 rabbits in the blank group and 162 rabbits in the poisoning group. The 162 rabbits in the poisoning group were randomly divided into a model group, D3 high-dose group, D3 low-dose group, compound 3B high-dose group, compound 3B low-dose group, composition 3 high-dose group, composition 3 low-dose group, composition 3-powder injection high-dose group, composition 3-powder injection low-dose group, with 18 rabbits in each group. Rabbits in the mode group and the blank group were administered by intravenous drip with the same volume of normal saline, with a rate of 30 drops/min for a dripping time of about 2h, 1 time/d, 5 d/week. The grouping and administration dose are shown in Table 5. After administration on the last day of week 1, week 2 and week 4, the urine of 0h~6h and the urine of 7h~24h of rabbits were respectively collected; the content of cadmium in blood, urine and kidney, and the cadmium-removing rate from blood and kidney were determined, and the results are shown in Table 6.

**Table 5: Grouping and administration dose of New Zealand rabbits in Example 18**

| Group | Name | Quantity | Administration and dose | |
|---|---|---|---|---|
| | | | Normal Saline | GMDTC preparation |
| A | Blank group | 18 | moderate | / |
| B | Model group | 18 | moderate | / |
| C-1 | D3 low-dose group | 18 | / | 108mg/kg |
| C-2 | D3 high-dose group | 18 | / | 433mg/kg |
| D-1 | Compound 3B low-dose group | 18 | / | 21mg/kg |
| D-2 | Compound 3B high-dose group | 18 | / | 83.5mg/kg |
| E-1 | Composition 3 low-dose group | 18 | / | 27mg/kg |
| E-2 | Composition 3 high-dose group | 18 | / | 108mg/kg |
| F-1 | Composition 3-preparation low-dose group | 18 | / | 27mg/kg |
| F-2 | Composition 3-preparation high-dose group | 18 | / | 108mg/kg |

**Table 6: Cadmium-removing rates (x̅ ± s) from blood and kidney of model rabbits**

| Group | Quantity | Duration of treatment (w) | Cadmium in blood (µg/L) | Removing rate (%) | Cadmium in kidney(µg/L) | Removing rate (%) |
|---|---|---|---|---|---|---|
| A | 6 | - | 1.30±0.43** | - | 0.81±0.32** | - |
| B | 6 | - | 103.61±11.16 | - | 11.76±3.28 | - |
| C-1 | 6 | 1 | 96.60±6.31 | 6.8 | 7.42±3.83* | 39.7 |
| C-2 | 6 | 1 | 89.45±13.31 | 13.9 | 6.53±2.16** | 47.8 |
| D-1 | 6 | 1 | 102.21±16.38 | - | 12.03±3.84 | - |
| D-2 | 6 | 1 | 101.54±13.27 | - | 11.94±3.51 | - |
| E-1 | 6 | 1 | 95.53±7.16 | 7.9 | 7.13±3.19* | 42.3 |
| E-2 | 6 | 1 | 89.92±10.35 | 13.4 | 6.35±2.74** | 49.4 |
| F-1 | 6 | 1 | 93.72±5.13 | 9.7 | 7.21±2.37** | 41.5 |
| F-2 | 6 | 1 | 87.43±8.23* | 15.8 | 6.13±3.10** | 51.4 |
| A | 6 | 2 | 1.227±0.389** | - | 0.765±0.412** | - |
| B | 6 | 2 | 73.60±16.84 | - | 12.06±2.99 | - |
| C-1 | 6 | 2 | 68.54±13.39 | 0.07 | 5.92±2.01** | 54.0 |
| C-2 | 6 | 2 | 61.39±12.47 | 16.9 | 3.36±1.08** | 77.0 |
| D-1 | 6 | 2 | 72.02±16.92 | - | 12.57±3.37 | - |
| D-2 | 6 | 2 | 73.18±15.27 | - | 12.62±5.37 | - |
| E-1 | 6 | 2 | 62.35±9.37 | 15.5 | 5.54±1.67** | 58.1 |
| E-2 | 6 | 2 | 59.38±11.45 | 19.6 | 3.31±1.29** | 77.6 |
| F-1 | 6 | 2 | 60.70±18.52 | 17.8 | 5.44±1.47** | 59.0 |
| F-2 | 6 | 2 | 54.61±8.49* | 26.2 | 3.17±1.27** | 78.7 |
| A | 6 | 4 | 0.37±0.41** | - | 0.77±0.25** | - |
| B | 6 | 4 | 27.35±12.16 | - | 13.83±3.27 | - |
| C-1 | 6 | 4 | 24.34±8.38 | 11.1 | 3.93±1.16** | 75.8 |
| C-2 | 6 | 4 | 17.67±6.84 | 35.8 | 1.85±0.52** | 91.7 |
| D-1 | 6 | 4 | 28.53±12.62 | - | 13.22±4.20 | - |
| D-2 | 6 | 4 | 27.92±14.72 | - | 14.02±2.96 | - |
| E-1 | 6 | 4 | 21.43±7.89 | 21.9 | 3.15±1.58** | 81.8 |
| E-2 | 6 | 4 | 16.75±7.45* | 39.3 | 1.56±0.98** | 94.0 |
| F-1 | 6 | 4 | 20.29±9.16 | 26.1 | 3.03±0.49** | 82.7 |
| F-2 | 6 | 4 | 16.11±5.30* | 41.6 | 1.41±0.35** | 95.1 |

In this and subsequent tables, * indicates p<0.05 compared with the model group at the same treatment time; ** indicates p<0.01 compared with the model group at the same treatment time.

Through the determination of the cadmium content in kidney, it can be seen that compared with the model group at the same treatment time, at the first week of administration, the cadmium contents in kidney of D3 low-dose group, D3 high-dose group, composition 3 low-dose group, composition 3 high-dose group, composition 3-preparation low-dose group, composition 3-preparation high low-dose group all significantly decreased, all with statistical differences compared with the model group; cadmium-removing rates of D3 high-dose group, composition 3 high-dose group and composition 3-preparation high-dose group were 47.8%, 49.4% and 51.4%, respectively. At the second week of administration, the cadmium-removing rates of D3 high-dose group, composition 3 high-dose group and composition 3-preparation high-dose group were 77.0%, 77.6% and 78.8%, respectively. At the fourth week of administration, the cadmium-removing rates of D3 high-dose group, composition 3 high-dose group and composition 3-preparation high-dose group were 91.7%, 94.0% and 95.1%, respectively. The dose of composition 3 and composition 3-preparation were both only 1/4 of the dose of compound D3 in patent CN200510035377.1, but the cadmium-removing rates were comparable. However, there was no significant difference between compound 3B with the model group, indicating that compound 3B had no cadmium-removing effect.

At the same dose of 108mg/kg, composition and composition-preparation had significantly higher 4-week cadmium-removing rate than compound D3.

We further tested urine β₂-microglobulin of D3 low-dose group, D3 high-dose group, compound 3B low-dose group, compound 3B high-dose group, composition 3 low-dose group, composition 3 high-dose group, composition 3-preparation low-dose group, and composition 3-preparation high-dose group after four weeks of administration, as shown in Table 7.

**Table 7: Urine β₂-microglobulin values (x̅ ± s) of each group after four weeks of administration**

| Group | β₂-microglobulin values(ng/L) |
|---|---|
| A | 100.62±12.35** |
| B | 205.14±45.17 |
| C-1 | 197.15±31.66 |
| C-2 | 161.64±45.18 |
| D-1 | 213.56±31.76 |
| D-2 | 209.42±38.21 |
| E-1 | 182.18±36.28 |
| E-2 | 146.15±20.42** |
| F-1 | 155.15±24.17* |
| F-2 | 105.15±19.74** |

Through the detection of the renal injury marker β₂-microglobulin, high-dose of composition 3 and composition 3-preparation could both effectively reduce the content of β₂-microglobulin, indicating that the kidney injury was repaired.

Therefore, composition 3 and composition 3-preparation can reduce the drug dose without affecting the efficacy, thereby reducing the exposure of the drug in the human body, reducing the risk of drug use, and repairing kidney damage. Secondly, due to the reduction of drug dose, the drug cost and the burden of patients can be effectively reduced.

### Example 19

The cadmium-removing effects of the oral preparations of composition 3 were studied in this embodiment.

According to the preparation methods in Examples 10, 11, 13, a batch of oral preparations were prepared, and the cadmium-removing effects were detected by animal experiments.

A total of 48 male New Zealand rabbits were randomly divided into 6 rabbits in a blank control group and 42 rabbits in chronic cadmium poisoning group. The 42 rabbits were ear margin intravenous injected with a mixed solution containing 1.5µmol/kg CdCl₂ and 30µmol/kg thioethanol (ME) according to weight, once/d for continuous 5 days, and observed for 35 days. After successful modeling, the 42 rabbits were randomly divided into 18 rabbits in a model group, 6 rabbits in enteric-coated granule low-dose group, 6 rabbits in enteric-coated granule high-dose group, 6 rabbits in enteric-coated capsule low-dose group, 6 rabbits in enteric-coated capsule high-dose group, 6 rabbits in enteric-coated sustained-release capsule low-dose group, and 6 rabbits in enteric-coated sustained-release capsule high-dose group; the administration last for continuous 14 days, the low dose was 300 mg/day and the high dose was 900 mg/day. The two-week cadmium-removing rates of high-dose enteric-coated granule/enteric-coated capsule/enteric-coated sustained-release capsule were 45.0%, 53.6% and 66.0%, respectively, as shown in Table 8.

**Table 8: Cadmium-removing rates (x̅ ± s) from kidney after administration of oral preparations for continuous 14 days**

| Group | Quantity | Dose(mg/kg) | Cadmium in kidney (µg/L) | Removing rate (%) |
|---|---|---|---|---|
| Blank group | 6 | - | 0.782±0.385** | - |
| Model group | 6 | - | 12.365±3.852 | - |
| Enteric-coated granule | 6 | 300 | 9.128±2.426 | 27.9 |
| Enteric-coated granule | 6 | 900 | 7.145±2.953** | 45.0 |
| Enteric-coated capsule | 6 | 300 | 7.831±3.425* | 30.5 |
| Enteric-coated capsule | 6 | 900 | 7.120±2.864** | 45.3 |
| Enteric-coated sustained-release capsule | 6 | 300 | 7.783±3.138** | 39.5 |
| Enteric-coated sustained-release capsule | 6 | 900 | 5.726±2.584** | 66.0 |

It can be seen from Table 8 that the oral preparations of composition 3 have a certain cadmium-removing effect, and the effect of sustained-release capsule is better than that of enteric-coated granule and enteric-coated capsule. It demonstrates that the longer the drug action time, the better the cadmium-removing effect.

### Example 20

The cadmium-removing effect of composition 3-transdermal preparation prepared in Example 14 was studied in this embodiment.

24 male New Zealand rabbits were randomly divided into 6 rabbits in a blank group and the remaining 18 rabbits. The 18 rabbits were ear margin intravenous injected with a mixed solution containing 1.5µmol/kg CdCl₂ and 30µmol/kg thioethanol (ME) according to weight, once/d for continuous 5 days, and observed for 49 days. After successful modeling, the 18 rabbits were divided into 6 rabbits in a model group, 6 rabbits in low-dose group and 6 rabbits in high-dose group. All rabbits were stripped of abdominal hair; rabbits in the blank group and control group were pasted with a transdermal preparation without composition 3, with a specification 4cm × 4cm; rabbits in low-dose group were pasted with a transdermal preparation containing composition 3, with a specification of 4cm × 4cm; rabbits in high-dose group were pasted with a transdermal preparation containing composition 3, with a specification of 8cm × 8cm; the transdermal preparations were changed twice a day, once in the morning and once in the evening, for 28 consecutive days. Urine was collected on day 28 for detection of urinary cadmium and β₂-microglobulin; rabbits were sacrificed on day 29, kidneys were taken and kidney cadmium was detected, as shown in Table 9.

**Table 9: Therapeutic effects of composition 3-transdermal preparations after treatment of 28 continuous days (x̅ ± s)**

| Group | Cadmium in urine(ng) | β₂-microglobulin(ng/L) | Cadmium in kidney(µg/g) |
|---|---|---|---|
| Blank group | 0.10±0.05** | 112.38±17.83** | 0.41±0.27** |
| Model group | 118.37±54.84 | 213.09±38.83 | 12.36±3.85 |
| low-dose group | 1374.37±369.48** | 157.19±35.05 | 9.26±3.39* |
| high-dose group | 1525.28±468.17** | 132.58±45.63** | 4.82±2.15** |

It can be seen from Table 9 that the levels of urine cadmium in high-dose and low-dose of composition 3-transdermal preparation groups increased significantly compared with that of the model group, and the levels of kidney cadmium in high-dose and low-dose of composition 3-transdermal preparation groups decreased significantly compared with that of the model group; indicating that composition 3-transdermal preparation has a significant cadmium-removing effect and presents a dose-dependent relationship. Moreover, β₂-microglobulin in high-dose group decreased significantly compared with that of the model group, indicating that kidney injury was recovered.

### Example 21

The therapeutic effect of composition 3-powder injection (referred to as composition 3-preparation) prepared in Example 7 on mercury poisoning was studied in this embodiment.

30 rabbits weighing 1.5~2.0 kg, half male and half female, were subcutaneously injected with 1% mercuric chloride every day, 0.8 mL each rabbit, for 3 continuous days; β₂-microglobulin increased significantly, indicating the formation of kidney damage symptoms and successful modeling.

After successful modeling, these rabbits were divided to 6 rabbits in the blank group and the remaining 24 rabbits; the 24 rabbits were randomly divided into 6 rabbits in a model group, 6 rabbits in composition 3-preparation low-dose group, 6 rabbits in 3-preparation middle-dose group, and 6 rabbits in composition 3-preparation high-dose group. Rabbits in the blank group and model group were administered by intravenous drip with normal saline, and the normal saline solution containing corresponding drug was administered to rabbits in the treating groups; rabbits in all groups were administered with the same volume and the same dripping rate of 30 drops/min, and the dripping time was about 2 h, 1 time/day, 5 days/week, for a total of 4 weeks. The grouping and administration dose are shown in Table 10, blood and urine were collected on days 0, 1, 5, 12, 19 and 26, respectively, blood mercury concentration and urine mercury concentration were detected, urine mercury content was calculated, and urine β₂-microglobulin was detected. Blood mercury concentrations are shown in Table 11, urine mercury contents are shown in Table 12, and results of urine β₂-microglobulin are shown in Table 13.

**Table 10: Grouping and administration dose of New Zealand rabbits in Example 21**

| Group | Name | Quantity | Administration and dose | |
|---|---|---|---|---|
| | | | Normal saline | GMDTC preparation |
| A | Blank group | 6 | moderate | / |
| B | Model group | 6 | moderate | / |
| C | Composition 3-preparation low-dose group | 6 | / | 12mg/kg |
| D | Composition 3-preparation middle-dose group | 6 | / | 36mg/kg |
| E | Composition 3-preparation high-dose group | 6 | / | 108mg/kg |

**Table 11: Blood mercury concentrations (ng/ml) in different groups (x̅ ± s)**

| Group | 0 day | 1 day | 5 days | 12 days | 26 days |
|---|---|---|---|---|---|
| A | 12±6** | 11±7** | 12±5** | 12±6** | 11±7** |
| B | 5358±2218 | 5187±2107 | 4218±1836 | 3617±1587 | 2419±895 |
| C | 5329±2579 | 4918±2716 | 3900±1898 | 3088±1260 | 1829±1222 |
| D | 5279±2618 | 4729±2238 | 3116±1604 | 1584±1118* | 719±348** |
| E | 5423±2364 | 4541±2010 | 2238±1292* | 819±479** | 486±398** |

**Table 12: 24-hour total urine mercury (ng) in different groups (x̅ ± s)**

| Group | 0 day | 1 day | 5 days | 12 days | 26 days |
|---|---|---|---|---|---|
| A | 1.51±0.34** | 1.47±0.28** | 1.37±0.25** | 1.33±0.31** | 1.26±0.15** |
| B | 13708±4079 | 14796±5043 | 15359±3390 | 14018±5516 | 13842±6828 |
| C | 14147±6590 | 17074±7788 | 17582±7245 | 18397±8916 | 38942±11524* |
| D | 13593±8721 | 188567±59985** | 199636±77488** | 209686±68682** | 189424±74128** |
| E | 14050±7466 | 527859±109786** | 632708±150231** | 480914±110479** | 48158±19684* |

**Table 13: Urinary β₂-microglobulin values (x̅ ± s) in different groups after administration of four weeks**

| Group | β₂-microglobulin(ng/L) |
|---|---|
| A | 107.72±16.09** |
| B | 433.79±68.02 |
| C | 352.56±94.05 |
| D | 231.16±41.19** |
| E | 177.78±36.15** |

From the above experimental results, it can be seen that, in the middle-dose and high-dose groups, the blood mercury concentration significantly decreased compared with that of the model group, and the urinary mercury excretion significantly increased compared with that of the model group, indicating that composition 3-preparation can increase the mercury excretion; further, β₂-microglobulin content significantly decreased, indicating that composition 3-preparation can treat the kidney damage caused by mercury.

### Example 22

The therapeutic effect of composition 3-powder injection prepared in Example 7 (referred to as composition 3-preparation) on lead poisoning was studied in this embodiment.

30 SD rats with a body weight of 180~200g were taken; rats in the blank group freely drank deionized water containing 12.5µL acetic acid for 3 weeks. Rats in lead poisoning group freely drank an aqueous solution containing 2.5 g/L lead acetate (containing 12.5µL/L acetic acid) for 3 weeks, resulting in experimental lead poisoning in rats. The lead content in the whole blood of rats in the blank group was (35.16 0.78) µg/L. The blood lead content of rats infected with lead for 3 weeks was (2459.12±38.27) µg/L (P<0.01), indicating that the lead absorbed through intestine had caused lead poisoning in rats.

After successful molding, 6 rats were divided in a blank group, and 30 rats were in divided into 5 groups. Rats in all groups were administrated once a day for 5 days/week for a total of 4 weeks. Rats in the blank group and model group were administered by intravenous drip with normal saline, and rats in treating groups were administered with normal saline solution of the corresponding drug; the administration volume of all groups was the same, with a dripping rate of 30 drops/min for a time of about 1h, 1 time/day, 5 days/week; the grouping and administration dose of rats are shown in Table 14.

On the last day, rats were sacrificed 24h after administration, blood, kidney, brain and liver were taken, and ICP-MS was used to detect the lead content in these organs, and the test results are shown in Table 15; the concentrations of calcium, magnesium, iron, copper and zinc in blood were also detected, and the test results are shown in Table 16.

**Table 14: Grouping and administration dose of SD rats in Example 22**

| Group | Name | Quantity | Administration and dose | | |
|---|---|---|---|---|---|
| | | | Normal saline | EDTA | Composition 3-preparation |
| A | Blank group | 6 | moderate | / | / |
| B | Model group | 6 | moderate | / | / |
| C | EDTA group | 6 | / | 93.5mg/kg | / |
| D | Composition 3-preparation low-dose group | 6 | / | / | 12mg/kg |
| E | Composition 3-preparation middle-dose group | 6 | / | / | 36mg/kg |
| F | Composition 3-preparation high-dose group | 6 | / | / | 108mg/kg |

**Table 15: Lead content in blood and organs in different group (x̅ ± s)**

| Group | Lead in blood (µg/L) | Lead in kidney (µg/g) | Lead in liver (µg/g) | Lead in brain (µg/g) |
|---|---|---|---|---|
| A | 35.16 ±0.78**^{ΔΔ} | 0.30±0.16**^{ΔΔ} | 0.06±0.03**^{ΔΔ} | 0.10±0.02**^{ΔΔ} |
| B | 2459.12±138.27 | 25.27±5.32 | 0.55±0.42 | 0.82±0.15 |
| C | 1218.69±129.33** | 16.54±4.67 | 0.47±0.28 | 0.52±0.11 |
| D | 1998.08±139.23*^{Δ} | 16.92±5.61 | 0.46±0.30 | 0.64±0.16 |
| E | 1476.93±162.14** | 8.16±3.53**^{ΔΔ} | 0.44±0.27 | 0.52±0.08 |
| F | 757.91±134.05**^{ΔΔ} | 4.05±2.31**^{ΔΔ} | 0.41±0.26 | 0.49±0.11* |

| | | | | |
|---|---|---|---|---|
| △ indicates that p<0.05 compared with the EDTA group at the same treatment time, and △△ indicates that p<0.01 compared with the EDTA group at the same treatment time. | | | | |

**Table 16: Blood concentrations of calcium, magnesium, iron, copper and zinc (x̅ ± s) in different groups**

| Group | Mg²⁺ (µg/g) | Ca²⁺ (µg/g) | Fe³⁺ (µg/g) | Cu²⁺ (µg/g) | Zn²⁺ (µg/g) |
|---|---|---|---|---|---|
| A | 42.94±5.93 | 90.19±6.47 | 184.17±27.44 | 618.64±96.71 | 3578.29±230.45 |
| B | 43.91±7.80 | 94.16±10.40 | 190.36±36.63 | 823.57±437.36 | 3386.51±438.11 |
| C | 43.61±3.32 | 89.31±5.28 | 194.72±19.29 | 754.21±70.59 | 2942.15±176.08*^{ΔΔ} |
| D | 46.51±1.81 | 82.72±4.17^{Δ} | 211.19±12.60 | 726.52±177.57 | 3588.12±189.81 |
| E | 44.15±4.50 | 92.41±5.80 | 208.26±12.39 | 740.28±186.55 | 3466.06±284.90 |
| F | 41.63±2.29 | 88.91±9.64 | 184.77±19.22 | 764.99±346.70 | 3316.42±297.43 |

| | | | | | |
|---|---|---|---|---|---|
| △ indicates p<0.05 compared with the blank group at the same treatment time; △△ indicates p<0.01 compared with the blank group at the same treatment time. | | | | | |

From the above experimental results, it can be seen: compared with the model group, the blood lead concentration in middle-dose and high-dose groups of composition 3-preparation and EDTA group significantly decreased, indicating that EDTA and composition 3-preparation could both reduce blood lead content, but high-dose composition 3-preparation had a significantly better effect than EDTA; compared with the model group and the EDTA group, the kidney lead concentration in middle-dose and high-dose groups of composition 3-preparation significantly decreased, indicating that composition 3-preparation can increase the excretion of kidney lead. In addition, at this dose, there was no significant difference between the levels of calcium, magnesium, iron, copper and zinc in composition 3-preparation group and the blank group/the model group, indicating that composition 3-preparation had no effect on calcium, magnesium, iron, copper and zinc in the blood at this experimental dose.

### Example 23

The therapeutic effect of composition 3-powder injection prepared in Example 7 (referred to as composition 3-preparation) on arsenic poisoning was studied in this embodiment.

60 Wistar rats with a body weight of 180~200 g were taken; rats in a blank group freely drank deionized water for 3 months; rats in arsenic poisoning groups freely drank an aqueous solution containing 100 mg/L·As₂O₃ for continuous 3 months, with a water mount of 20 ml per day, equivalent to an oral dose of 10 mg/kg, resulting in arsenic poisoning of drinking water-type in rats.

After successful molding, rats were divided to 10 rats in blank group and the remaining 50 rats. All groups were administrated once a day for 5 days/week for a total of 4 weeks. Rats in the blank group and the model group were administered by intravenous drip with normal saline, and rats in the treating group were administered with a normal saline solution of the corresponding drug; all groups had the same administration volume, a drip rate of 30 drops/min, and an dripping time of about 1h, 1 time/d, 5d/week, for a total of four weeks. The grouping and administration dose of Wistar rats in different groups are shown in Table 17.

Urine was collected on day 1 and day 26, arsenic excretion was measured; blood and kidney of rats were taken 24 hours after the 26th day of administration, and blood arsenic concentrations and renal arsenic concentrations were detected with ICPMS; the results are shown in Table 18.

**Table 17: Grouping and administration dose of Wistar rats in Example 23**

| Group | Name | Quantity | Administration and dose | | |
|---|---|---|---|---|---|
| | | | Normal saline | EDTA | GMDTC preparation |
| A | Blank group | 10 | moderate | / | / |
| B | Model group | 10 | moderate | / | / |
| C | EDTA group | 10 | / | 93.5mg/kg | / |
| D | Composition 3-preparation low-dose group | 10 | / | / | 12mg/kg |
| E | Composition 3-preparation middle-dose group | 10 | / | / | 36mg/kg |
| F | Composition 3-preparation high-dose group | 10 | / | / | 108mg/kg |

**Table 18: Arsenic concentrations in blood and kidney and arsenic contents in urine in different groups (x̅ ± s)**

| Group | Arsenic in blood (µg/L) | Arsenic in kidney (µg/g) | Total arsenic in urine on day 1 (µg) | Total arsenic in urine on day 26 (µg) |
|---|---|---|---|---|
| A | 23.79 ±2.96** | 1.57±0.93** | 0.06±0.01** | 0.07±0.01** |
| B | 3596.17±893.46 | 6.52±1.83 | 1.52±0.43 | 1.14±0.33 |
| C | 1362.98±319.58** | 6.08±2.35 | 3.27±1.84** | 3.18±1.35** |
| D | 2817.21±616.01 | 6.16±1.89 | 2.18±1.47 | 3.49±1.32** |
| E | 1527.97±359.33** | 4.49±1.27** | 4.52±2.15** | 4.94±1.83** |
| F | 849.98±258.57** | 1.12±0.54** | 8.32±3.18** | 3.68±1.59* |

From the above experimental results, it can be seen that compared with the model group, arsenic concentrations in blood and kidney can be significantly reduced by middle-dose and high-dose of composition 3-preparation; arsenic content in urine was significantly increased by middle-dose and high-dose of composition 3-preparation on day 1, indicating that composition 3-preparation has a significant promoting effect on the excretion of arsenic.

### Example 24

The therapeutic effect of composition 3-powder injection prepared in Example 7 (referred to as composition 3-preparation) on thallium poisoning was studied in this embodiment.

60 Wistar rats with a body weight of 180~200 g were taken; rats in a blank group freely drank deionized water for 1 months; rats in thallium poisoning group freely drank an aqueous solution containing 40 mg/L·Tl₂SO₄ for continuous 1 month, with a water mount of 20 ml per day, equivalent to an oral dose of 4 mg/kg, resulting in thallium poisoning of drinking water-type in rats.

After successful molding, rats were divided to 10 rats in the blank group and the remaining 50 rats, and the grouping and administration dose are shown in Table 19. Rats in all groups were administrated once a day for 5 days/week for a total of 4 weeks. Rats in the blank group and the model were administered by intravenous drip with normal saline, and rats in the treating groups were administered with a normal saline solution of the corresponding drug; all groups had the same administration volume, with a drip rate of 30 drops/min, and an instillation time of about 1h, once/d, 5d/week, for a total of four weeks.

Rats were sacrificed 24h after administration on the last day; blood, kidney, brain and liver were taken, and thallium contents in these organs were detected by ICP-MS, and the test results are shown in Table 20.

**Table 19: Grouping and administration dose of Wistar rats in Example 24**

| Group | Name | Quantity | Administration and dose | | |
|---|---|---|---|---|---|
| | | | Normal saline | EDTA | GMDTC preparation |
| A | Blank group | 10 | moderate | / | / |
| B | Model group | 10 | moderate | / | / |
| C | EDTA group | 10 | / | 93.5mg/kg | / |
| D | Composition 3-preparation low-dose group | 10 | / | / | 12mg/kg |
| E | Composition 3-preparation middle-dose group | 10 | / | / | 36mg/kg |
| F | Composition 3-preparation high-dose group | 10 | / | / | 108mg/kg |

**Table 20: Thallium contents in blood and organs (x̅ ± s) in different groups**

| Group | Thallium in blood (µg/L) | Thallium in kidney (µg/g) | Thallium in liver (µg/g) | Thallium in brain (µg/g) |
|---|---|---|---|---|
| A | 0.00±0.00** | 0.00±0.00** | 0.00±0.00** | 0.00±0.00** |
| B | 1269.45±248.38 | 15.51±7.94 | 5.73±1.15 | 1.23±0.26 |
| C | 836.69±168.83 | 12.54±4.48 | 4.27±2.18 | 1.13±0.11 |
| D | 984.26±135489 | 13.92±3.72 | 5.46±2.37 | 1.17±0.16 |
| E | 493.60±197.57** | 9.16±2.29** | 4.44±1.18* | 0.98±0.18 |
| F | 268.14±115.70** | 2.05±1.27** | 3.41±1.06** | 1.09±0.11 |

From the above experimental results, it can be seen that, compared with the model group, middle-dose and high dose of composition 3-preparation can significantly reduce the concentration of blood thallium, kidney thallium and liver thallium; while EDTA cannot reduce thallium concentration in kidney and liver; indicating that composition 3-preparation has a significant promoting effect on the excretion of thallium.

### Example 25

The therapeutic effect of composition 3-powder injection prepared in Example 7 (referred to as composition 3-preparation) on copper poisoning was studied in this embodiment.

30 SD rats with a weight of 80~100 g were divided into three groups: A, B and C. Group A is a blank group and rats in group A ate freely. Groups B and C were copper poisoning groups, and 1.60 g/kg of CuSO₄ was added to the feed; rats in group B and group C ate freely in an SPF environment every day for 2 months, resulting in rat copper poisoning models.

After successful modeling, rat in all groups were administrated once a day for 5 days/week, for a total of 4 weeks. Rats in group A and group B were administered intraperitoneally with normal saline; rats in group C were administered intraperitoneally with a normal saline solution containing 433 mg/kg composition 3-preparation. Rats in all groups were administered with the same volume, once/d for 5 days. 24 hour-urine was collected after administration every day, copper concentration in urine was detected by ICP-MS, and the excretion amount was calculated, as shown in Figure 2.

It can be seen from FIG. 2 that composition 3-preparation can significantly increase the excretion of copper ions at a dose of 433 mg/kg.

### Example 26

The therapeutic effect of composition 3-powder injection prepared in Example 7 (referred to as composition 3-preparation) on mixed poisoning of chromium, cobalt, nickel, and manganese.

40 New Zealand rabbits with a weight of 1.5~2.0kg were taken, and normal saline was injected intravenously at the ear margin of rabbits of the blank group for 5 days. A mixed solution containing 5 mg/ml CrCl₃, 3 mg/ml CoCl₂, 1 mg/ml NiCl₂ and 2 mg/ml MnCl₂ was injected intravenously at the ear margin of rabbits of poisoning groups with a dose of 2 ml/kg for 5 days, and observed for 25 days, thus resulting in mixed poisoning models of chromium, cobalt, nickel and manganese in New Zealand rabbits.

After successful molding, there were 8 rabbits in the blank group and 24 rabbits in mixed poisoning groups. Rabbits in all groups were given once daily for 3 days. Rabbits in the blank group and the model group were administered by intravenous dripping with normal saline, and rabbits in the treating groups were administered with a normal saline solution containing the corresponding drug. The administration volume of all groups was the same, with a dripping rate of 30 drops/min, an administration time of about 2h, 1 time/day for 3 days; the grouping and administration dose of New Zealand rabbit in different groups were shown in Table 21. 24-hour urine was collected every day for a total of 3 days, and the total amounts of chromium, cobalt, nickel and manganese excreted through urine during the 3 days were calculated, as shown in Table 22.

**Table 21: Grouping and administration dose of New Zealand rabbits in Example 26**

| Group | Name | Quantity | Administration and dose | | |
|---|---|---|---|---|---|
| | | | Normal saline | EDTA | GMDTC preparation |
| A | Blank group | 8 | moderate | / | / |
| B | Model group | 8 | moderate | / | / |
| C | EDTA group | 8 | / | 93.5mg/ kg | / |
| D | Composition 3-preparation group | 8 | / | / | 216mg/kg |

**Table 22: Total amounts of chromium, cobalt, nickel and manganese excreted through urine (x̅ ± s) in New Zealand rabbits after 3 days of treatment**

| Group | Chromium in urine (µg) | Cobalt in urine (µg) | Nickel in urine (µg) | Manganese in urine (µg) |
|---|---|---|---|---|
| A | 0.57 ±0.18**^{ΔΔ} | 0.67±0.23**^{ΔΔ} | 0.51±0.23**^{ΔΔ} | 0.40±0.17**^{ΔΔ} |
| B | 12.64±8.52 | 8.86±5.11 | 9.71±5.255 | 9.54±3.86 |
| C | 178.48±42.69** | 162.28±44.97** | 188.28±63.17** | 169.52±32.63** |
| D | 326.97±185.45**^{Δ} | 498.21±188.48**^{ΔΔ} | 683.29±210.30**^{ΔΔ} | 434.82±197.36**^{ΔΔ} |

| | | | | |
|---|---|---|---|---|
| △ indicates p<0.05 compared with EDTA group at the same treatment time, and △△ indicates p<0.01 compared with EDTA group at the same treatment time. | | | | |

From the above experimental results, it can be seen that compared with the model group and EDTA group, the total amount of chromium, cobalt, nickel and manganese excreted through urine in composition 3-preparation group significantly increased, indicating that composition 3-preparation has a promoting effect on the excretion of chromium, cobalt, nickel and manganese, and is superior than EDTA.

### Example 27

The tin-removing effect and protective effect against oxidative damage caused by a tin-containing compound of composition 3-powder injection prepared in Example 7 (referred to as composition 3-preparation) were studied in this embodiment.

36 ICR mice were randomly divided into three groups, 12 in each group; mice in the blank group were intraperitoneally injected with normal saline, mice in the model group and the treating group were intraperitoneally injected with 0.5 mg/kg trimethyltin chloride; after three days of infection, mice in the blank group and the model group were administrated with normal saline, and mice in the treating group were administrated with a normal saline solution containing 252.2mg/kg composition 3-preparation; intraperitoneal injection for 3 days was adopted for all the groups. Three days of urine was collected, the total amount of urine tin was measured; mice were sacrificed three days later, mouse livers were taken and stored in liquid nitrogen, and reactive oxygen species (ROS) and malondialdehyde (MDA) content were detected; the test results are shown in Table 23.

**Table 23: Contents of urine tin, ROS and MDA (x̅ ± s) in different groups of mice**

| Group | Day 1, urine tin (ng) | Day 2, urine tin (ng) | Day 3, urine tin (ng) | ROS (U/mg) | MDA (nmol/mg) |
|---|---|---|---|---|---|
| Blank group | 2.1±0.3** | 2.0±0.3** | 2.1±0.4** | 780.5±110.4** | 1.34±0.23** |
| Model group | 35.7±10.5 | 39.2±11.2 | 45.2±14.5 | 1440.8±137.3 | 2.65±0.31 |
| Drug group | 144.2±35.6** | 175.3±42.7** | 162.5±31.4** | 785.2±109.5** | 1.33±0.18** |

From the above experimental results, it can be seen that compared with the model group, the treatment group showed a significant increase in urinary tin excretion, indicating that composition 3-preparation can effectively promote tin excretion; meanwhile, levels of ROS and MDA significantly decreased, and the oxidative stress response in the body was restored. Therefore, composition 3-preparation has a promoting effect on tin excretion and a protective effect against oxidative damage caused by tin-containing compounds.

### Example 28

The scavenging effect on free radicals of composition 3-powder injection prepared in Example 7 (referred to as composition 3-preparation) was studied in this embodiment.

40 Wistar rats, with a body weight of 200 ~ 230g, half male and female, were placed in a clean-grade animal chamber, weighed, numbered, and randomly divided into groups A, B, C, and D with a toal of 40 rats. D-galactose injection was prepared with normal saline at a concentration of 5%, rats in groups B, C and D were injected subcutaneously with D-galactose 500 mg/(kg·d) at the abdomen to prepare aging models, and rats in group A (the blank group) were injected with the same dose of normal saline at the abdomen; the injection lasted for 56 days. On the first day after successful modeling, rats in C group and D group were intraperitoneally administrated with 108 mg/kg and 216 mg/kg composition 3-preparation, and rats in A group and B group were administrated with 10 mL/kg normal saline; diet and water were free, and the administration lasted for continuous 30 days. 8 h after the last administration, anesthesia was conducted by intraperitoneal administration of 3% pentobarbital sodium, the abdomen was exposed, blood was taken from the inferior vena cava, after centrifugation, the supernatant was taken as the serum to be measured; serum hydroxyl radicals (OH- • ) clearance, superoxide dismutase (SOD), glutathione peroxidase (GSH-PX), and malondialdehyde (MDA) were detected, as shown in Table 24.

**Table 24: (OH⁻) clearance, contents of SOD, GSH-PX and MDA of rats in different groups (x̅ ± s)**

| Group | OH⁻·Clearance | SOD activity (U/mg) | GSH-PX activity (U/mg) | MDA content (nmol/mg) |
|---|---|---|---|---|
| A | / | 74.83±12.44** | 89.29±12.47** | 48.35±12.82** |
| B | / | 51.36±13.47 | 66.54±15.32 | 78.19±13.19 |
| C | 23.45% | 64.73±10.56* | 78.19±13.67* | 58.97±12.38* |
| D | 39.43% | 70.54±10.88** | 85.18±12.92** | 50.46±11.59** |

From the above experimental results, it can be seen that compared with model B group, the SOD and GSH-PX activities of the treatment groups (C and D) significantly increased, and the MDA content significantly decreased; indicating that composition 3-preparation has obvious scavenging effect on free radicals in vivo.

### Example 29

The bismuth-removing effect of composition 3-powder injection prepared in Example 7 (referred to as composition 3-preparation) was studied in this embodiment.

40 Wistar rats with a body weight of 180~200g were taken; rats in the blank group freely drank deionized water for 1 month. Rats in the bismuth poisoning group freely drank an aqueous solution containing 100 mg/L bismuth potassium citrate, with a drinking amount of about 20 ml of water every day, for continuous 3 months, resulting in bismuth poisoning in rats.

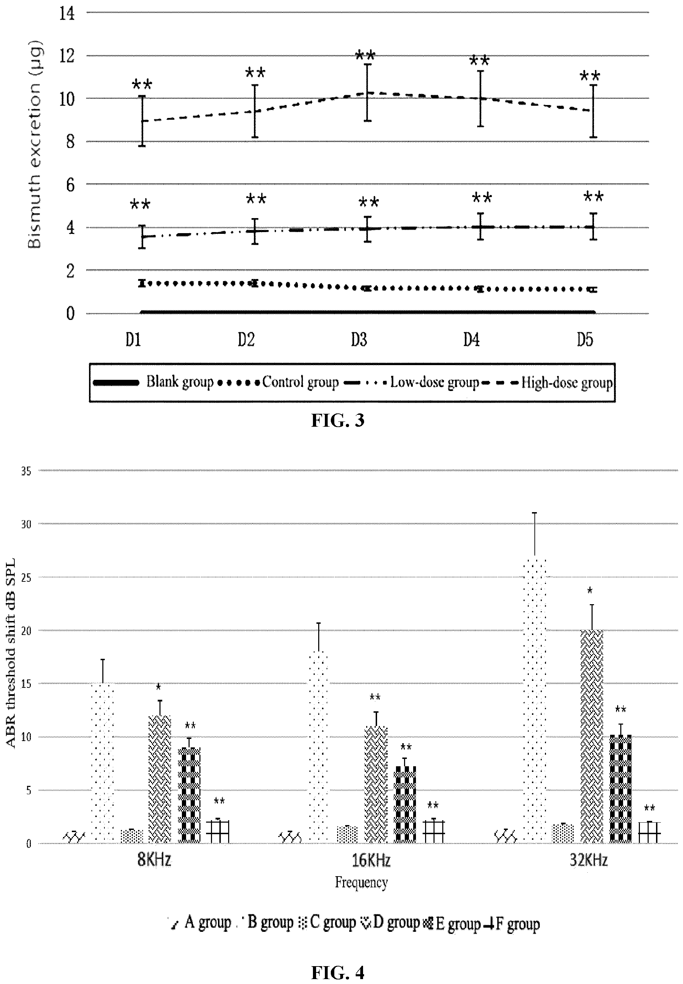

After successful modeling, 10 rats were selected into the blank group and 30 rats were selected into the poisoning group, and rats in the poisoning group was divided into a control group, a low-dose group and a high-dose group, with 10 animals in each group; rats in all groups were administered once a day for 5 consecutive days. Rats in the blank group and the control group were injected intraperitoneally with normal saline, rats in the low-dose group were administrated with a normal saline solution containing 27mg/kg composition 3-preparation, rats in the high-dose group were administrated with a normal saline solution containing 108 mg/kg composition 3-preparation. Rats in all groups were administered by intraperitoneal injection with the same volume, 1 time/day, 5 days/week; daily urine was collected to detect bismuth concentration, and the daily excretion of bismuth was calculated, as shown in Figure 3.

It can be seen from Figure 3 that compared with the control group, high-dose and low-dose of composition 3-preparation can both significantly increase the excretion of urine bismuth, and there is a dose-response relationship.

### Example 30

The effect of composition 3-powder injection prepared in Example 7 (referred to as composition 3-preparation) to reduce cisplatin-induced ototoxicity was studied in this embodiment.

60 SPF grade NIH mice were selected, and after 3 days of quarantine, they were randomly divided into 6 groups, grouping and administration are shown in Table 25; mice in all groups were administered with the same dosage volume by intraperitoneal injection, 1 time/day, 5 days/week, for a total of 2 weeks. The timing of administration is shown in Table 26. Before and after intraperitoneal injection, auditory brainstem response (ABR) test was performed on each mouse by the "intelligent EP" auditory evoked potential diagnosis system to evaluate hearing function.

**Table 25: Grouping and administration of NIH mouse in Example 30**

| Group | Name | Quantity | Administration and dose of administration | | |
|---|---|---|---|---|---|
| | | | Normal saline | Cisplatin | Composition 3-preparation |
| A | Blank group | 10 | moderate | / | / |
| B | Cisplatin group | 10 | moderate | 7.5mg/kg | / |
| C | Composition 3-preparation group | 10 | / | / | 216mg/kg |
| D | Composition 3-preparation low-dose group | 10 | / | 7.5mg/kg | 54mg/kg |
| E | Composition 3-preparation middle-dose group | 10 | / | 7.5mg/kg | 108mg/kg |
| F | Composition 3-preparation high-dose group | 10 | / | 7.5mg/kg | 216mg/kg |

**Table 26: Administration schedule of mice in different groups in Example 30**

| Group | Day 1 | Day 2-5 | Day 6-7 | Day 8-12 |
|---|---|---|---|---|
| A | Normal saline | Normal saline | / | Normal saline |
| B | Cisplatin | Normal saline | / | Normal saline |
| C | Composition 3-preparation | Composition-3 preparation | / | Composition 3-preparation |
| D | Cisplatin + 2 hours later composition 3-preparation | Composition-3 preparation | / | Composition 3-preparation |
| E | Cisplatin + 2 hours later composition 3-preparation | Composition-3 preparation | / | Composition 3-preparation |
| F | Cisplatin + 2 hours later composition 3-preparation | Composition-3 preparation | / | Composition 3-preparation |

Mice were anesthetized by intraperitoneal injection with ketamine (90 mg/kg) before administration and after 12 days of administration, tested in a controlled acoustic chamber, where the grounding electrode was located on the posterior side, the positive electrode was inserted directly between the ears at the top of the skull, and the negative electrode was located below the auricle; through a high-frequency probe, the sound stimulation intensity started from 80dB SPL at frequencies of 8, 16, and 32KHz, and gradually decreases by 5dB. The difference in response threshold of the same stimulus frequency before and after administration was recorded as ABR threshold shift. The results are shown in Figure 4.

It can be seen from Figure 4 that there was no significant difference between composition 3-preparation group and the blank group, indicating that composition 3-preparation itself does not cause ototoxicity, and the combination therapy groups were significantly different from cisplatin group, indicating that the combination therapy can significantly reduce cisplatin-induced ototoxicity, and there is a dose-dependent relationship.

### Example 31

The attenuating effect of composition 3 prepared in Example 1 on cisplatin was studied in this embodiment.

40 SPF grade New Zealand rabbits, 4-8 weeks old, 1.8-2.2 kg, with standard diet and free acess to water, were randomly divided into A, B, C, D four groups. Group A was a blank group, and rats in group A were injected with 0.9% sodium chloride injection through the ear vein. Group B was the cisplatin group, and rats in group B were injected with 4mg/kg cisplatin injection through the ear vein twice a week, and injected with normal saline for 3 days in the remaining days of a week. Group C was cisplatin-D3 group, and rats in group C were injected with 4mg/kg cisplatin injection through the ear vein twice a week, and injected with 433mg/kg D3 through ear vein dripping 5 times a week. Group D was cisplatin-composition 3 group, and rats in group D were injected with 4mg/kg cisplatin injection through the ear vein twice a week, and injected with 433mg/kg composition 3 through ear vein dripping 5 times a week. All New Zealand rabbits were administered for 3 weeks, 5 days a week, and behavioral activities thereof were observed. The grouping and administration dose are shown in Table 27, and the administration schedule is shown in Table 28.

**Table 27: Grouping and administration dose in Example 31**

| Group | Name | Quantity | Administration and dose | | |
|---|---|---|---|---|---|
| | | | Normal saline | Cisplatin | Composition 3 |
| A | Blank group | 10 | moderate | / | / |
| B | Cisplatin group | 10 | moderate | 4mg/kg | / |
| C | Cisplatin-D3 group | 10 | / | 4mg/kg | 433mg/kg |
| D | Cisplatin-composition 3 group | 10 | / | 4mg/kg | 433mg/kg |

**Table 28: Administration schedule in Example 31**

| Group | Day 1-2, 8-9, 15-16 | Day 3-5, 10-12, 17-19 | Day 6-7, 13-14 |
|---|---|---|---|
| A | Normal saline | Normal saline | / |
| B | Cisplatin | Normal saline | / |
| C | Cisplatin + D3 | D3 | / |
| D | Cisplatin + Composition 3 | Composition 3 | / |

Experimental results: in group B, after 1 week, rabbits in this group experienced weight loss, hair removal, and loose stools; during 2 weeks, all rabbits experienced a weight loss of more than 20% of the body weight, significant emaciation, malaise, hair removal, loose stools, nasal bleeding, oral bleeding, and weak breathing, and all rabbits reached ethical endpoints and were euthanized. In group C, after 1 week, rats in this group experienced weight loss, hair removal, loose stools, nasal bleeding, and oral bleeding; during 2^{nd} and 3^{rd} weeks, all rabbits experienced a weight loss of more than 20% of the body weight, significant emaciation, malaise, and weak breathing, and all rabbits reached ethical endpoints and were euthanized. In group D, after 3 weeks of administration, rats in this group experienced a weight loss of 5% -15% and mild emaciation; after discontinuing the administration, weight began to recover.

The above experimental results show that at the same dose, the attenuating effect of composition 3 on cisplatin is better than D3.

### Example 32

The effect of composition 3 prepared in Example 1 to reduce the side effects of radioactive elements on the nude mouse pancreatic cancer model was studied in this embodiment.

Pancreatic cancer model nude mice, 4 weeks old, 16-20g, were selected, anesthetized, monitored for 14 days; tumor size were detected; mice were randomly divided into A, B, C, D, E 5 groups according to tumor size and body weight. Mice in group A received free diet; mice in group B and group C were implanted with iodine-125; mice in group D and group E were implanted with palladium-103. Mice in group B and group D were administrated with 108mg/kg composition 3 per day for 14 consecutive days, and tumor size was detected on day 0, 7 and 14, respectively, as shown in Table 29. On day 14, urine and blood were collected and radiation intensity was measured using a liquid scintillation counter.

Liquid scintillation count results: the blood radiation intensity of group B was less than that of group C, p<0.05; the urine radiation intensity of group B was greater than that of group C; the blood radioactivity intensity of group E was greater than that of group D, p<0.05; the urine radiation intensity of group E was less than that of group D.

**Table 29: Tumor volumes cm³(x±s) of nude mice**

| Group | Day 0 | Day 7 | Day 14 |
|---|---|---|---|
| A | 0.28±0.02 | 0.65±0.06 | 1.45±0.10 |
| B | 0.27±0.03 | 0.22±0.03** | 0.16±0.03** |
| C | 0.27±0.02 | 0.23±0.03** | 0.15±0.03** |
| D | 0.28±0.02 | 0.19±0.03** | 0.10±0.03** |
| E | 0.29±0.02 | 0.18±0.04** | 0.10±0.03** |

From the above results, it can be seen that composition 3 does not affect the antitumor effect of iodine-125 and palladium-103. Moreover, composition 3 can complex with iodine-125 and palladium-103, and excreted from the body.

### Example 33

The effect of composition 3 prepared in Example 1 on Tc excretion was studied in this embodiment.

16 New Zealand rabbits weighing 1.5~2.0 kg were taken and divided into two groups, A and B; rabbits in group A received intravenous bolus of sodium high-Tc [99mTc] acid injection and administration of normal saline three hours later; mice in group B received intravenous bolus of sodium high-Tc [99mTc] acid injection and administration of 433 mg/kg composition 3 three hours later. All groups received administration of the same volume, with a drip rate of 15 drops/min and an infusion time of about 3h for each time. Urine of the first 12 hours was collected, Tc concentration in urine was measured by ICP-MS, and the total Tc excretion was calculated.

Experimental results: the urine excretion of technetium in group A was 42.3% of the injection amount, and the urinary excretion of technetium in group B was 74.5% of the injection amount; indicating that composition 3 could significantly increase the excretion of technetium, and compared with group A, the urinary excretion of technetium increased by 76.1%.

### Example 34

The therapeutic effect of an antitumor medicament (composed of composition 3-powder injection (referred to as composition 3-preparation) prepared in Example 7 and cisplatin) on rabbit VX2 liver tumor animal model was studied in this embodiment.

SPF grade New Zealand rabbits, 4-8 weeks old, 1.8-2.2kg, with a standard diet and free access to water, were kept in an environment of 12-hour light/dark cycle with a constant temperature of (23 ± 2C) and a humidity of (55 ± 15%). The animals were anesthetized, guided by ultrasound, and VX2 hepatoma cells were introduced into the liver by minimally invasive approach, After monitoring New Zealand rabbits for 14 days, and tumor size was monitored with ultrasound; rabbits were randomly divided into 4 groups of 10 animals in each group according to tumor size and body weight, and drug treatment was started on day 15. Rabbits in the control group were intravenously injected with 0.9% sodium chloride injection through the ear vein. Rabbits in cisplatin group were intravenously injected with 4 mg/kg cisplatin through the ear vein once a week. Rabbits in treatment group A (cisplatin: composition 3-preparation in the drug combination = 1:20) were intravenously injected with 4 mg/kg cisplatin through the ear vein once a week, and injected with 60 mg/kg composition 3-preparation 2 hours after cisplatin injection. Rabbits in treatment group B (cisplatin: composition 3-preparation in drug combination = 1: 100) were intravenously injected with 4mg/kg cisplatin through the ear vein once a week, and injected with 400mg/kg composition 3-preparation 2 hours after cisplatin injection. All New Zealand rabbits were treated for 4 weeks.

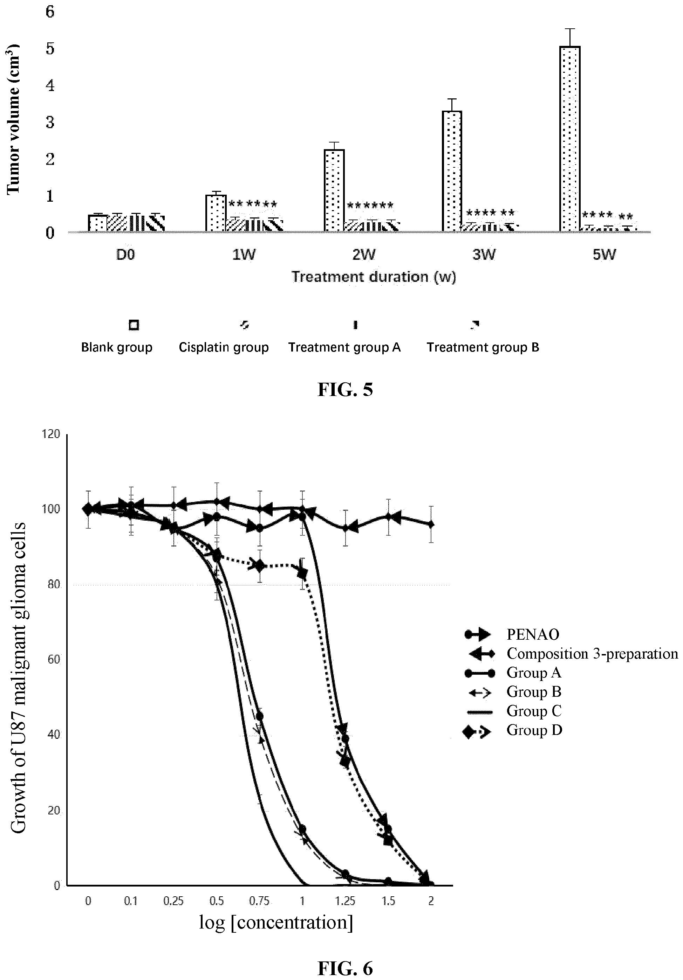

Tumor volume was estimated using the formula V= π (a × b²)/6, where a and b were the longest and shortest diameters of the tumor measured using calipers. The weights of rabbits were monitored every 3-4 days during the experiment, and rabbited were sacrificed at the end of treatment; blood was collected, AST, ALT and CREA were tested, and the results are shown in Table 30. After anesthetizing each rabbit with Sutai 50+ tachypnosis 0.1 ml/kg, carotid artery phlebotomy was used as a termination procedure. In addition, tumor was taken and the tumor volume was recorded immediately after sacrifice, as shown in Figure 5.

**Table 30: AST, ALT, CREA detection values (x ± s) of rabbits in different groups**

| Group | ALT(U/L) | AST(U/L) | CREA(µmol/L) |
|---|---|---|---|
| Blank | 67.02±38.29 | 52.90±26.86** | 82.28±20.88** |
| Cisplatin | 86.68±26.88 | 89.20±13.66 | 470.35±85.57 |
| Treatment group A | 71.56±24.37 | 59.32±12.81** | 128.26±6.46** |
| Treatment group B | 54.88±18.71* | 40.55±13.32** | 82.60±7.33** |

From the above experimental results, it can be seen that the antitumor medicament composed of cisplatin and composition 3-preparation had no obvious difference in tumor inhibition compared with cisplatin, both the two could effectively inhibit the growth of tumor cells and kill tumor cells. Compared with cisplatin alone, the combination group significantly reduced the hepatotoxicity and nephrotoxicity caused by cisplatin, and the reduction of hepatotoxicity and nephrotoxicity caused by cisplatin in treatment group B was superior to that in treatment group A.

### Example 35

The inhibiting effect of an antitumor medicament (composed of composition 3-powder injection (referred to as composition 3-preparation) prepared in Example 7 and PENAO (structural formula below)) on tumor was studied in this embodiment. 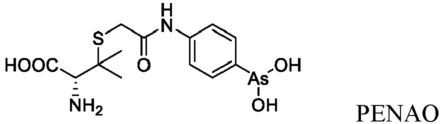
Group A: PENAO: composition 3-preparation = 1: 20,
Group B: antineoplastic drugs are PENAO: composition 3-preparation = 1: 50,
Group C: antineoplastic drugs are PENAO: composition 3-preparation = 1: 125,
Group D: antineoplastic drugs are PENAO: composition 3-preparation = 1: 312.

IC₅₀ values of PENAO/composition 3-preparation/antitumor medicament (composed of PENAO and composition 3-preparation) on U87 malignant glioma cells were tested; different groups had the same original concentration of drug. The results are shown in Figure 6.

It can be seen from Figure 6: composition 3-preparation has no obvious inhibitory effect on U87 malignant glioma cells; PENAO had obvious inhibitory effect on U87 malignant glioma cells; the antitumor medicament composed of PENAO and composition 3-preparation had a significantly enhanced inhibitory effect on glioma cells, which was superior to PENAO alone.

### Example 36

The cadmium-removing effects of compositions 1-11 were studied in this embodiment.

150 male New Zealand rabbits, 1.8~2.2 kg/pc, were randomly divided into 15 rabbits in blank control group and 135 in chronic cadmium poisoning model group. The model rabbit were intravenous injected at ear margin with a mix solution containing 1.5µmol/kg CdCl₂ and 30µmol/kg thioethanol (ME) according to weight, once/d for continuous 5 days, and observed for 35 days. After 35 days of observation, the qualified rabbits were selected according to their weight, including 10 rabbits in the blank group and 120 rabbits in poisoning groups; the poisoning rabbits were randomly divided into 10 rabbits in a model group, 10 rabbits in each group of compositions 1~11; rabbits in the blank group and model group were administered by intravenous drip with the same volume of normal saline, 30 drops/min, and the instillation time was about 2h, 1 time/d, 5 d/week, for a total of 2 weeks; rabbits in the treating groups received the administration with a dose of 0.25mmol/kg, a rate of 30 drops/min, a instillation time of about 2h, and a frequency of 1 time/d and 5d/week for a total of 2 weeks. The content of cadmium in the kidney was detected after 2 weeks, and the results are shown in Table 31.

**Table 31: Cadmium-removing effects from kidney of composition 1-11 at the same administration concentration**

| Group | Administration dose (mmol/kg) | Content of cadmium in kidney | Cadmium-removing rate from kidney (%) |
|---|---|---|---|
| Blank group | / | 0.65±0.32** | / |
| Model group | / | 12.13 ±3.11 | / |
| Composition 1 | 0.25 | 7.13 ±2.14* | 43.55 |
| Composition 2 | 0.25 | 6.76±3.49* | 55.49 |
| Composition 3 | 0.25 | 3.45±2.01** | 75.61 |
| Composition 4 | 0.25 | 4.98±2.74** | 62.28 |
| Composition 5 | 0.25 | 5.92±2.47** | 54.09 |
| Composition 6 | 0.25 | 7.79±3.26* | 37.80 |
| Composition 7 | 0.25 | 7.61±3.42* | 39.37 |
| Composition 8 | 0.25 | 5.49±2.73** | 57.84 |
| Composition 9 | 0.25 | 5.17±2.18** | 60.63 |
| Composition 10 | 0.25 | 5.51±3.66** | 57.67 |
| Composition 11 | 0.25 | 5.67±3.05** | 56.27 |

Through the determination of cadmium content in kidney, it can be seen that compared with the model group at the same treatment time, when administered for 2 weeks, composition 1~11 had better cadmium-removing effects from rabbit kidney, and the cadmium-removing effect of composition 3 was higher than that of other 10 compounds.

It is necessary to explain the following points: (1) the existing drugs to removing heavy metals, such as EDTA complexing agents, dimercaptopropanol, and sodium dimercaptosulphonate can not drive away heavy metals in kidney, and these substances will even increase the burden on kidney. Therefore, although cadmium-removing effects from kidney of compositions 1, 6 and 7 are significantly lower than that of other compositions, compositions 1, 6 and 7 still have clinical value. (2) The efficacy results provided in the present embodiment are completed with specific experimental animals and under specific administration conditions. Although cadmium-removing effects from kidney of compositions 1, 6 and 7 are significantly lower than that of other compositions, prolonging the administration time and increasing the administration amount could improve their cadmium-removing effects from kidney; in addition, the ability of drugs to removing cadmium from kidney may vary between species. (3) Compounds 1, 6 and 7 have good lipid solubility, which is conducive to intestinal absorption when prepared into oral preparations, and have advantages in bioavailability.

### Example 37

Compositions 1-11 and compounds 1A-11A were placed at environments of -25°C, 5 ± 3°C, 25 ± 2 °C /60 ± 5%RH, 40 ± 2°C /75 ± 5% RH, 60°C for 0d, 5d and 10d (d refers to day), respectively, and changes of the main component content (the main component content of 0 day was converted to 100%) were measured, the results are shown in Table 32.

**Table 32: Changes of the main component content of compositions 1-11 and compounds 1A-11A over time under different conditions**

| SN | Condition | Group | 0d | 5d | 10d |
|---|---|---|---|---|---|
| 1 | 60°C | Compound 1A | 100% | 59.4% | 58.7% |
| | | Composition 1 | 100% | 87.9% | 86.7% |
| | 40±2°C/ 75±5%RH | Compound 1A | 100% | 83.5% | 81.5% |
| | | Composition 1 | 100% | 97.7% | 96.9% |
| | 25±2°C/ 60±5%RH | Compound 1A | 100% | 97.3% | 92.1% |
| | | Composition 1 | 100% | 98.9% | 98.4% |
| | 5±3°C | Compound 1A | 100% | 99.5% | 99.1% |
| | | Composition 1 | 100% | 100.7% | 100.0% |
| | -25°C | Compound 1A | 100% | 100.2% | 100.1% |
| | | Composition 1 | 100% | 100.3% | 100.1% |
| 2 | 60°C | Compound 2A | 100% | 49.9% | 48.8% |
| | | Composition2 | 100% | 67.4% | 66.2% |
| | 40±2°C/ 75±5%RH | Compound 2A | 100% | 94.1% | 90.1% |
| | | Composition2 | 100% | 99.9% | 96.2% |
| | 25±2°C/ 60±5%RH | Compound 2A | 100% | 91.4% | 82.5% |
| | | Composition2 | 100% | 97.3% | 93.7% |
| | 5±3°C | Compound 2A | 100% | 99.5% | 98.1% |
| | | Composition2 | 100% | 100.7% | 100.0% |
| | -25°C | Compound 2A | 100% | 100.2% | 99.8% |
| | | Composition2 | 100% | 101.0% | 100.3% |
| 3 | 60°C | Compound 3A | 100% | 65.6% | 64.2% |
| | | Composition3 | 100% | 95.5% | 94.2% |
| | 40±2°C/ 75±5%RH | Compound 3A | 100% | 91.2% | 84.2% |
| | | Composition3 | 100% | 96.4% | 95.4% |
| | 25±2°C/ 60±5%RH | Compound 3A | 100% | 95.1% | 90.3% |
| | | Composition3 | 100% | 99.4% | 97.3% |
| | 5±3°C | Compound 3A | 100% | 97.3% | 96.5% |
| | | Composition3 | 100% | 100.4% | 99.7% |
| | -25°C | Compound 3A | 100% | 101.2% | 99.8% |
| | | Composition 3 | 100% | 100.3% | 101.2% |
| 4 | 60°C | Compound 4A | 100% | 68.0% | 64.7% |
| | | Composition 4 | 100% | 93.2% | 92.1% |
| | 40±2°C/ 75±5%RH | Compound 4A | 100% | 83.4% | 80.8% |
| | | Composition 4 | 100% | 99.7% | 99.3% |
| | 25±2°C/ 60±5%RH | Compound 4A | 100% | 92.8% | 91.7% |
| | | Composition 4 | 100% | 98.9% | 96.6% |
| | 5±3°C | Compound 4A | 100% | 97.6% | 95.3% |
| | | Composition 4 | 100% | 99.8% | 100.2% |
| | -25°C | Compound 4A | 100% | 100.2% | 100.5% |
| | | Composition 4 | 100% | 100.5% | 100.2% |
| 5 | 60°C | Compound 5A | 100% | 80.3% | 78.7% |
| | | Composition 5 | 100% | 95.2% | 94.1% |
| | 40±2°C/ 75±5%RH | Compound 5A | 100% | 97.9% | 91.8% |
| | | Composition 5 | 100% | 99.9% | 99.9% |
| | 25±2°C/ 60±5%RH | Compound 5A | 100% | 97.8% | 95.7% |
| | | Composition 5 | 100% | 99.9% | 99.6% |
| | 5±3°C | Compound 5A | 100% | 99.6% | 98.7% |
| | | Composition 5 | 100% | 100.6% | 101.3% |
| | -25°C | Compound 5A | 100% | 100.7% | 101.1% |
| | | Composition 5 | 100% | 101.1% | 100.2% |
| 6 | 60°C | Compound 6A | 100% | 60.4% | 57.8% |
| | | Composition 6 | 100% | 86.8% | 75.3% |
| | 40±2°C/ 75±5%RH | Compound 6A | 100% | 91.5% | 91.0% |
| | | Composition 6 | 100% | 99.5% | 97.5% |
| | 25±2°C/ 60±5%RH | Compound 6A | 100% | 93.1% | 85.3% |
| | | Composition 6 | 100% | 97.9% | 94.2% |
| | 5±3°C | Compound 6A | 100% | 98.8% | 99.1% |
| | | Composition 6 | 100% | 100.1% | 100.0% |
| | -25°C | Compound 6A | 100% | 100.6% | 99.9% |
| | | Composition 6 | 100% | 100.3% | 100.5% |
| 7 | 60°C | Compound 7A | 100% | 80.2% | 80.1% |
| | | Composition 7 | 100% | 98.4% | 97.9% |
| | 40±2°C/ 75±5%RH | Compound 7A | 100% | 98.7% | 96.5% |
| | | Composition 7 | 100% | 100.1% | 98.9% |
| | 25±2°C/ 60±5%RH | Compound 7A | 100% | 99.3% | 98.0% |
| | | Composition 7 | 100% | 100.4% | 101.0% |
| | 5±3°C | Compound 7A | 100% | 100.0% | 99.1% |
| | | Composition 7 | 100% | 101.3% | 100.5% |
| | -25°C | Compound 7A | 100% | 100.1% | 100.6% |
| | | Composition 7 | 100% | 101.0% | 100.5% |
| 8 | 60°C | Compound 8A | 100% | 80.4% | 79.7% |
| | | Composition 8 | 100% | 94.8% | 94.3% |
| | 40±2°C/ 75±5%RH | Compound 8A | 100% | 94.7% | 89.6% |
| | | Composition 8 | 100% | 99.9% | 99.9% |
| | 25±2°C/ 60±5%RH | Compound 8A | 100% | 95.5% | 93.3% |
| | | Composition 8 | 100% | 98.5% | 96.9% |
| | 5±3°C | Compound 8A | 100% | 97.9% | 96.2% |
| | | Composition 8 | 100% | 100.2% | 100.3% |
| | -25°C | Compound 8A | 100% | 101.1% | 100.3% |
| | | Composition 8 | 100% | 100.4% | 101.0% |
| 9 | 60°C | Compound 9A | 100% | 84.5% | 79.3% |
| | | Composition 9 | 100% | 97.9% | 95.7% |
| | 40±2°C/ 75±5%RH | Compound 9A | 100% | 92.6% | 91.5% |
| | | Composition 9 | 100% | 98.3% | 97.3% |
| | 25±2°C/ 60±5%RH | Compound 9A | 100% | 98.9% | 96.0% |
| | | Composition 9 | 100% | 100.6% | 100.1% |
| | 5±3°C | Compound 9A | 100% | 97.2% | 96.0% |
| | | Composition 9 | 100% | 100.4% | 100.4% |
| | -25°C | Compound 9A | 100% | 100.7% | 100.5% |
| | | Composition 9 | 100% | 100.3% | 100.2% |
| 10 | 60°C | Compound 10A | 100% | 82.0% | 79.1% |
| | | Composition 10 | 100% | 95.5% | 94.6% |
| | 40±2°C/ 75±5%RH | Compound 10A | 100% | 92.0% | 90.3% |
| | | Composition 10 | 100% | 98.9% | 97.0% |
| | 25±2°C/ 60±5%RH | Compound 10A | 100% | 97.0% | 96.1% |
| | | Composition 10 | 100% | 99.8% | 99.0% |
| | 5±3°C | Compound 10A | 100% | 99.5% | 98.1% |
| | | Composition 10 | 100% | 100.2% | 100.6% |
| | -25°C | Compound 10A | 100% | 100.5% | 101.4% |
| | | Composition 10 | 100% | 100.5% | 101.3% |
| 11 | 60°C | Compound 11A | 100% | 68.0% | 64.7% |
| | | Composition 11 | 100% | 91.1% | 91.0% |
| | 40±2°C/ 75±5%RH | Compound 11A | 100% | 88.10% | 88.5% |
| | | Composition 11 | 100% | 98.2% | 97.5% |
| | 25±2°C/ 60±5%RH | Compound 11A | 100% | 96.3% | 92.9% |
| | | Composition 11 | 100% | 99.6% | 98.4% |
| | 5±3°C | Compound 11A | 100% | 95.4% | 93.8% |
| | | Composition 11 | 100% | 100.4% | 99.7% |
| | -25°C | Compound11A | 100% | 100.9% | 99.8% |
| | | Composition 11 | 100% | 100.7% | 100.2% |

With respect to the various temperature conditions in Table 32: the cost of cold chain storage and transportation corresponding to -25°C is high, the cost of cold chain storage and transportation corresponding to 5 °C is high, and the cost of normal temperature storage and transportation corresponding to 25 °C is low; 40°Cand 60°C are accelerated decomposition conditions that save experimental time, and drugs are not usually stored and transported at such temperatures. From the experimental results in Table 32, it can be seen that under the condition of 25 °C, the stability of each of compositions 1-11 was better than that of the corresponding compound selected from compounds 1-11A after 5d and 10d, indicating that the stability of each composition is better than that of a single compound at room temperature and within 10 days; under accelerated conditions (40°C and 60°C), the stability of each of compositions 1-11 was better than that of the corresponding compound selected from compounds 1-11A after 5d and 10d, indicating that the stability of each composition is better than that of a single compound at normal temperature for more than 10 days.

Although embodiments of the present disclosure have been shown and described above, it is understood that the above embodiments are exemplary and cannot be understood as limitations on the present invention, and those of ordinary skill in the art may make changes, modifications, substitutions, and variations to the above embodiments within the scope of the present disclosure without departing from the principles and purposes of the present disclosure. The scope of protection of the present disclosure is limited by the claims and their equivalent technical solutions.

## Claims

1. A highly stable heavy metal-removing composition, comprising:
(A) 70-95wt% of a compound represented by formula (1) or a pharmaceutically acceptable salt thereof;
(B) 1-25wt% of a compound represented by formula (2) or a pharmaceutically acceptable salt thereof;
(C) 0.001-5wt% of an alkaline compound, and the alkaline compound is at least one selected from the group consisting of a hydroxide of an alkali metal, a carbonate of an alkali metal, a bicarbonate of an alkali metal, a biphosphate of an alkali metal, a carboxylate of an alkali metal, and ammonium hydroxide;
wherein a combination of the compound represented by formula (1) and the compound represented by formula (2) is one selected from the combinations listed in the table below:
| SN | Formula (1) | Formula (2) |
|---|---|---|
| 1 | 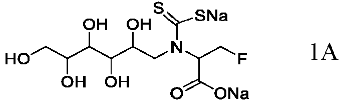 | 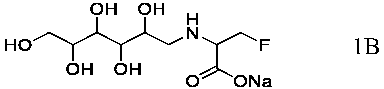 |
| 2 | 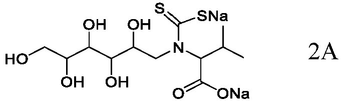 | 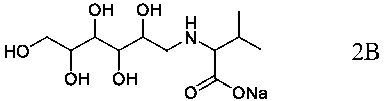 |
| 3 | 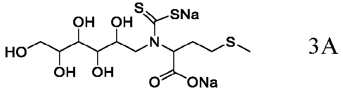 | 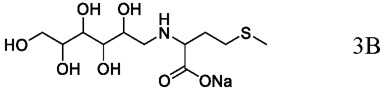 |
| 4 | 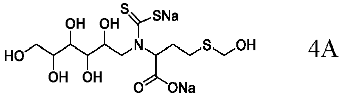 | 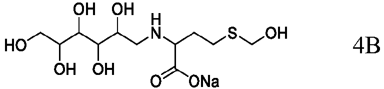 |
| 5 | 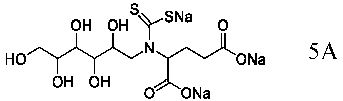 | 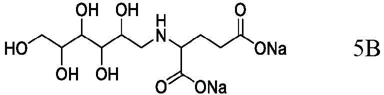 |
| | | |
|---|---|---|
| 6 | 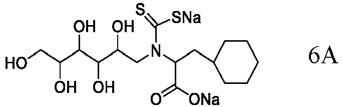 | 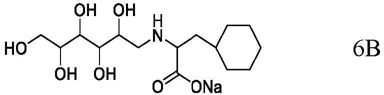 |
| 7 | 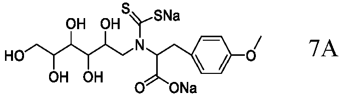 | 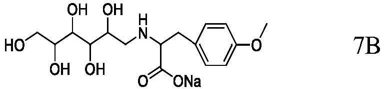 |
| 8 | 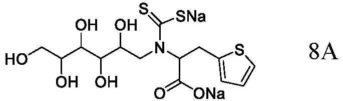 | 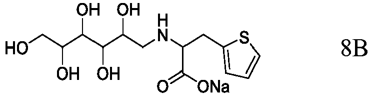 |
| 9 | 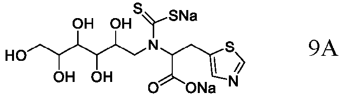 | 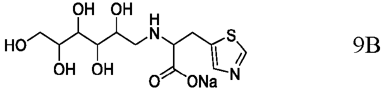 |
| 10 | 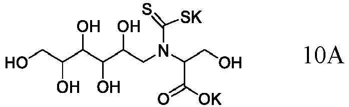 | 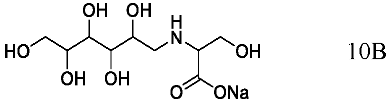 |
| 11 | 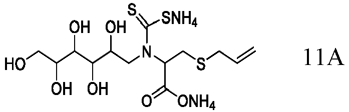 | 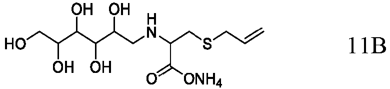 |

2. The composition according to claim 1, wherein the compound represented by formula (1) is 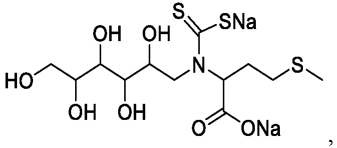 and
the compound represented by formula (2) is

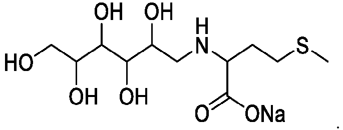

3. The composition according to claim 1, wherein a content of component (A) in the composition is 80-90wt%, and a content of component (B) in the composition is 1-12wt%.

4. The composition according to claim 1, wherein a ratio of a mass fraction of component (A) in the composition to a mass fraction of component (B) in the composition is in the range from 90:1 to 9:1.

5. The composition according to claim 4, wherein the ratio of the mass fraction of component (A) in the composition to the mass fraction of component (B) in the composition is in the range from 80:1 to 13:1.

6. The composition according to claim 1, wherein the alkaline compound is at least one selected from the group consisting of sodium hydroxide, potassium hydroxide, sodium acetate, ammonium hydroxide, sodium carbonate, sodium bicarbonate, ammonium bicarbonate, potassium carbonate, potassium bicarbonate, dipotassium hydrogenphosphate, and disodium hydrogenphosphate.

7. The composition according to claim 1, wherein a pH value of a solution formed by dissolving 1mg of the composition in 1mL of water is 9.0~11.0.

8. Use of the composition according to any one of claims 1-7 in the preparation of a medicament or a functional food for removing a heavy metal or free radicals from a body, or in the preparation of a medicament for preventing or treating a related disease caused by heavy metal-excess and heavy metal-poisoning, wherein the heavy metal is at least one selected from the group consisting of chromium, cobalt, arsenic, tin, cadmium, mercury, manganese, nickel, copper, thallium, technetium, uranium, bismuth, lead, iodine, palladium and platinum.

9. An antineoplastic medicament, comprising by weight: 1 portion of a heavy metal-containing antineoplastic drug, and 5-200 portions of the composition according to any one of claims 1-7.

10. The antineoplastic medicament according to claim 9, wherein the heavy metal-containing antineoplastic drug is selected from: a platinum-containing antineoplastic drug, a arsenic-containing antineoplastic drug, a ruthenium-containing antineoplastic drug or a tin-containing antineoplastic drug.

11. The antineoplastic medicament according to claim 9, wherein the heavy metal-containing antineoplastic drug is selected from: cisplatin, oxaliplatin, arsenic trioxide or iodine 125.

12. Use of the composition according to any one of claims 1-7 in the preparation of a medicament for reducing a toxic side effect of a heavy metal-containing drug, wherein the heavy metal-containing drug comprises at least one selected from the group consisting of cisplatin, carboplatin, oxaliplatin, nedaplatin, bismuth potassium citrate, colloidal pectin bismuth, technetium 99 dimethyl bisphosphonate, technetium 99mTc, arsenic trioxide, iodine 125 and palladium 103.

13. The use according to claim 12, wherein the heavy metal-containing drug comprises at least one selected from the group consisting of cisplatin, bismuth potassium citrate and technetium 99mTC.

14. A powder injection, wherein based on a total weight of the powder injection, the powder injection comprises:
60-90wt% of the composition according to any one of claims 1-7;
9-40wt% of an excipient; and
0.001-10wt% of a complexing agent.

15. The powder injection according to claim 14, wherein the excipient is at least one selected from the group consisting of mannitol, ammonium hydroxide, trehalose, sodium bicarbonate, sodium carbonate, potassium carbonate, sodium acetate, ammonium acetate and dipotassium hydrogenphosphate.

16. The powder injection according to claim 15, wherein the excipient is at least one selected from the group consisting of mannitol and ammonium hydroxide.

17. The powder injection according to claim 15, wherein the excipient is ammonium hydroxide, and ammonium hydroxide is present in the form of a complex or ammine in the powder injection.

18. The powder injection according to any one of claims 14-17, wherein the complexing agent is at least one selected from the group consisting of ethylenediaminetetraacetate, dimercaptopropanol, and sodium dimercaptosuccinate.

19. The powder injection according to claim 18, wherein the complexing agent is ethylenediaminetetraacetate.

20. An oral preparation, wherein based on a total weight of the oral preparation, the oral preparation comprises:
35-65wt% of the composition according to any one of claims 1-7;
20-40wt% of a disintegrant;
0-5wt% of a flow aid;
0.1-5wt% of a lubricant;
0~10wt% of a film-coating premix; and
0.001-10wt% of a complexing agent.

21. The oral preparation according to claim 20, wherein the disintegrant is at least one selected from the group consisting of microcrystalline cellulose, sodium carboxymethylcellulose, sodium carboxymethyl starch, starch, and polyvinylpyrrolidone; the flow aid is at least one selected from the group consisting of talc, and colloidal silica; the lubricant is at least one selected from the group consisting of talc, and magnesium stearate; and the complexing agent is at least one selected from the group consisting of ethylenediaminetetraacetate, dimercaptopropanol, and sodium dimercaptosuccinate.

22. The oral preparation according to claim 21, wherein the disintegrant is microcrystalline cellulose, the flow aid is colloidal silica, the lubricant is magnesium stearate, and the complexing agent is ethylenediaminetetraacetate.

23. The oral preparation according to claim 22, wherein the oral preparation is an enteric-coated tablet or an enteric-coated capsule.

24. The oral preparation according to claim 23, wherein the enteric-coated tablet is an enteric-coated sustained-release tablet, and the enteric-coated capsule is an enteric-coated sustained-release capsule.

25. A transdermal preparation, wherein based on a total weight of the transdermal preparation, the transdermal preparation comprises:
20-65wt% of the composition according to any one of claims 1-7;
0.5-30wt% of a transdermal enhancer;
0.5-25wt% of a gelling agent; and
1-30wt% of a solvent.

26. The transdermal preparation according to claim 25, wherein the transdermal enhancer is at least one selected from the group consisting of laurocapram, borneol, oleic acid, and peppermint oil; the gelling agent is at least one selected from the group consisting of carbomer, sodium hydroxypropylcellulose, sodium ethylcellulose, magnesium stearate, glycerol and polyethylene glycol; and the solvent is at least one selected from the group consisting of water, methanol, ethanol and DMSO.

27. A method for preparing the composition according to any one of claims 1-7, wherein the method is carried out under a protection of an inert gas, and comprises the following steps:
(1) synthesis of component (B):
(1-1) adding and fully dissolving an alkaline compound, an amino acid and glucose in solvent A, and allowing to react to obtain a reaction product of (1-1);
(1-2) adding sodium borohydride to the reaction product of (1-1), allowing to react, then conducting acidification and purification to obtain a reaction product of (1-2);
(1-3) adding and fully dissolving an alkaline compound and the reaction product of (1-2) in solvent A, and allowing to react to obtain component (B);
(2) synthesis of component (A):
(2-1) dissolving an alkaline compound and the reaction product of (1-2) in water to obtain solution A;
(2-2) dissolving CS₂ in solvent B to obtain solution B;
(2-3) mixing solution A and solution B, allowing to react to obtain a reaction mixture, filtering the reaction mixture to obtain a filtrate, extracting the filtrate with solvent C to obtain a aqueous phase, and lyophilizing the aqueous phase to obtain a product;
wherein a mixing ratio of solution A and solution B is a ratio which makes the product consist of only component (A);
wherein solvent A is at least one selected from the group consisting of methanol, ethanol, acetone, acetonitrile, tetrahydrofuran and water; solvent B at least one selected from the group consisting of acetone, acetonitrile, tetrahydrofuran, dioxane and DMF; and solvent C is at least one selected from the group consisting of dichloromethane, ethyl acetate, propyl acetate, butyl acetate and isopropyl acetate; and
(3) mixing component (A), component (B) and an alkaline compound in proportion to obtain the composition.

28. A method for preparing the composition according to any one of claims 1-7, wherein the method is carried out under a protection of an inert gas, and comprises the following steps:
(1) synthesis of component (B):
(1-1) adding and fully dissolving an alkaline compound, an amino acid and glucose in solvent A, and allowing to react to obtain a reaction product of (1-1);
(1-2) adding sodium borohydride to the reaction product of (1-1), allowing to react, then conducting acidification and purification to obtain a reaction product of (1-2);
(1-3) adding and fully dissolving an alkaline compound and the reaction product of (1-2) in solvent A, and allowing to react to obtain component (B);
(2) synthesis of the composition:
(2-1) dissolving an alkaline compound and component (B) in water to obtain solution A;
(2-2) dissolving CS₂ in solvent B to obtain solution B; and
(2-3) mixing solution A and solution B, allowing to react to obtain a reaction mixture, filtering the reaction mixture to obtain a filtrate, extracting the filtrate with solvent C to obtain a aqueous phase, and lyophilizing the aqueous phase to obtain a product;
wherein a mixing ratio of solution A and solution B is a ratio which makes the product be the composition; and
wherein solvent A is at least one selected from the group consisting of methanol, ethanol, acetone, acetonitrile, tetrahydrofuran and water; solvent B at least one selected from the group consisting of acetone, acetonitrile, tetrahydrofuran, dioxane and DMF; and solvent C is at least one selected from the group consisting of dichloromethane, ethyl acetate, propyl acetate, butyl acetate and isopropyl acetate.

29. The method according to claim 28, wherein in step (2), a molar ratio of the alkaline compound to component (B) is 1.05: 1 to 1.5: 1, and a molar ratio of CS₂ to component (B) is 1.3: 1 to 5: 1.

30. The method according to claim 29, wherein the molar ratio of the alkaline compound to component (B) is 1.05: 1 to 1.3: 1, and the molar ratio of CS₂ to component (B) is 1.8: 1 to 3: 1.
